# EUROPEAN PATENT APPLICATION

(11) **EP 4 528 754 A1**
(43) Date of publication of application: **26.03.2025**
(21) Application number: 24201321.7
(22) Date of filing: 19.09.2024
(51) Int. Cl.: G21K 1/02, A61B 6/03

(54) **GAMMA RADIATION IMAGING**

(30) Priority: 22.09.2023 NL 2035862; 29.09.2023 NL 2035923; 29.09.2023 NL 2035924; 29.09.2023 NL 2035925
(71) Applicant: Free Bee International B.V., 2805 KH Gouda (NL)
(72) Inventor: BEEKMAN, Frederik Johannes, 2805 KH Gouda (NL)
(74) Representative: EP&C

(57) **Abstract**

A collimator part for in a gamma radiation imaging system, for example for scintimammography, comprises a plate with a planar incident surface at a front side and a rear surface at a rear side. A virtual normal plane extends perpendicular to the plate. Multiple inclined first and second holes extend through the plate from a position on the rear surface on a respective first or second side of the normal plane. Each of the first and second holes has a sight line extending along the hole from the rear surface to the planar incident surface and outwards beyond the planar incident surface. For at least one first hole and for at least one second hole a respective crossing position with the normal plane is positioned inward of the planar incident surface, e.g. in a groove at the front side.

## Description

The inventions described herein relate to the field of gamma radiation imaging. For example, the inventions are applied for scintimammography or imaging another human body part, e.g. an extremity like an arm or a leg, or imaging an animal body part, or another object.

### First invention

The first invention relates to a collimator part for use in the field of gamma radiation imaging, for example for scintimammography or for imaging another human body part, e.g. an extremity like an arm or a leg, or for imaging an animal body part.

Known systems for scintimammography relating to the first invention are described, for example, in WO2010008538, WO2010/014001, WO2010120636, US2013/0158389, and US105159456.

In gamma radiation imaging, a collimator part is used for forming an image on the detector of a gamma camera.

In a known embodiment, the collimator part is embodied as a plate comprising a planar incident surface at a front side and a rear surface at a rear side. A virtual normal plane extends perpendicular to the plate at a join between a first section of the plate and a second section of the plate. In the first section multiple inclined parallel first holes extend through the plate from a position on the rear surface on a first side of the normal plane. The first holes have an opening in the planar incident surface. In the second section multiple inclined parallel second holes extend through the plate from a position on the rear surface on a second side of the normal plane. The second holes have an opening in the planar incident surface. Each of the first and second holes has a sight line extending along the hole from the rear surface to the planar incident surface and outwards beyond the planar incident surface into an imaging space wherein the body part to be imaged is located. Gamma radiation passes from the body part through the first and second holes onto a detector of the gamma camera when an image is made. In the known collimator part all openings of the first holes are located on the one side of the normal plane and in the planar incident surface. All openings of the second holes are located on the other side of the normal plane and in the planar incident surface. Such a collimator part is known as a dual slant hole collimator. The field of view of this collimator part is the area where the sightlines of the first holes overlap with the sightlines of the second holes.

Because points in the body part to be imaged are covered by two sight lines, each under a different angle, such collimator parts provide good depth resolution. However, their field of view close to the collimator part is not satisfactory. For example, when the known collimator part is used for detecting a region(s) of interest, e.g. a tumorous lesion(s), in a human or animal body part, e.g. in a female breast, shallow lying region(s) are not imaged satisfactory. Herein, in practice, shallow lying means that the region is close to the collimator part during imaging, which commonly corresponds to close to the skin of the body part, e.g. the breast.

It is an object of the first invention to provide a collimator part which provides for an enhanced field of view, for example for use in scintimammography, e.g. in view of enhanced detection of region(s) of interest in shallow lying region(s).

In order to achieve at least one of the objects of the first invention, the first invention provides a collimator part according to claim 1.

In the inventive collimator part of the first invention a first sight line of at least one first hole crosses the normal plane on a first crossing position, a second sight line of at least one second hole crosses the normal plane on a second crossing position, and for at least one first hole and for at least one second hole the respective first crossing position and second crossing position is positioned inward of the planar incident surface. So, if the plate has parallel front and rear sides that define a thickness of the plate between them, there are in the collimator part at least one first hole and at least one second hole, in practice a set of multiple first holes and a set of multiple second holes, of which the respective crossing point lies within the thickness of the plate. This in contrast to the known collimator part discussed above, wherein all crossing points of the first and second holes with the normal plane are located outward of the front side of the collimator. In the inventive concept, in practical embodiments, a majority of the first and second holes will still be configured so that their crossing points are located outward of the front side of the collimator part. Yet, some of the first holes and second holes, in a zone which includes the normal plane, will have a crossing point with the normal plane inward of the planar incident surface.

Compared to the known collimator part there are now sight lines that cross the normal plane inward of the planar incident surface, which provides a larger and/or more effective field of view when it comes to effectively imaging also region(s) of interest that are located close to the incident surface of the collimator part, such as shallow lying tumours in breast tissue.

In a practical embodiment of the first invention, the collimator part is provided with an elongated recess at the front side which is recessed relative to the planar incident surface and which extends in the normal plane, wherein at least one first hole and at least one second hole adjoin the elongated recess, wherein for said first hole and second hole the respective first crossing position and second crossing position is positioned in the elongated recess, inward of the planar incident surface.

Preferably, the elongated recess at the front side is symmetrical relative to the normal plane.

In embodiments, the elongated recess is a groove in the front side of the front side of the collimator part, recessed relative to the planar incident surface, e.g. a V-shaped groove.

In a practical embodiment, the elongated recess is delimited by a first recess surface and a second recess surface at the first side and second side of the normal plane, respectively,
wherein, preferably, the first recess surface and the second recess surface are flat surfaces, e.g. to define a V-shaped groove,
- a first hole defines an opening in the first recess surface,
- a second hole defines an opening in the second recess surface,
- a first sight line of the at least one first hole is parallel to the second recess surface,
- a second sight line of the at least one second hole is parallel to the first recess surface.

Instead of providing an elongated recess, which appears the practically preferred embodiment to implement the inventive concept of the first invention, one could also arrive at the collimator part of claim 1 without providing the elongated recess. In this alternative design of the first invention, the planar incident surface remains a continuous surface. In a zone of the collimator part including the normal plane, at least one first hole and at least one second hole are arranged offset from one another in the direction of the intersecting line of the normal plane and the incident surface, wherein each of these holes extends from a location on the rear at one side of the normal plane to a location at the front side on the other side of the normal plane. So, their sight lines have a crossing point with the normal plane that is located inward of the planar incident surface. In practice, this collimator part will have a center zone where first holes and second holes alternate when looking in the mentioned direction along the mentioned intersecting line. This allows for a limited number of first and second holes, compared to the provision of the elongated recess as discussed herein.

In an embodiment, the first sight line of at least one first hole intersects the second sight line of at least one second hole at a point of intersection which is located in the elongated recess, wherein the point of intersection is, preferably, positioned on the normal plane.

In an embodiment, the inclined first holes are parallel to one another to form a first parallel slant hole collimator, and the inclined second holes are parallel to one another to form a second parallel slant hole collimator. In another embodiment, the first holes and second holes are configured in a fan beam arrangement, e.g. with the inclination of the holes increasing as they are further away from the normal plane.

In an embodiment, the sight lines of the inclined first holes are arranged at a first angle with respect to the normal plane and the sight lines of the inclined second holes are arranged at a second angle with respect to the normal plane, wherein the magnitude of the first angle is the same as the magnitude of the second angle. In other embodiments, the first and second angles differ from one another.

In embodiments, the first and second holes each have a uniform cross-section, e.g. diameter, over the length thereof.

The first invention also relates to a gamma camera comprising a detector for gamma radiation and a collimator part as discussed herein.

The first invention also relates to a gamma radiation imaging system, for example for scintimammography, comprising:
- a frame supporting a first immobilization plate and a second immobilization plate, which second immobilization plate is arranged or arrangeable to extend parallel to the first immobilization plate, wherein the first and second immobilization plates define an imaging space between them and are configured to clamp a body part to be imaged, e.g. a breast, between the immobilization plates,
- a first gamma camera configured and arranged to detect gamma radiation emitted from a volume in the imaging space passing through the first immobilization plate,
wherein the first gamma camera comprises:
- a first collimator assembly which extends in a plane parallel to the first immobilization plate, which first collimator assembly comprising a first collimator part as discussed herein in relation to the first invention,
- a first gamma sensitive detector arranged to receive gamma radiation passing through the first collimator assembly.

In an embodiment, the first collimator assembly further comprises a second collimator part, wherein the second collimator part is provided with parallel perpendicular holes which extend parallel to the normal plane.

In an embodiment, the system further comprises a second gamma camera configured and arranged to detect gamma radiation emitted from a volume in the imaging space passing through the second immobilization plate, wherein the second gamma camera comprises:
- a second collimator assembly, which extends in a plane parallel to the second immobilization plate,
- a second gamma sensitive detector arranged to receive gamma radiation passing through the second collimator assembly.

In an embodiment, the first and/or the second collimator assembly is movable relative to the respective immobilization plate in said plane in a displacement direction, preferably by a collimator motion device configured to controllably move the collimator assembly in the displacement direction.

In an embodiment, at least one of the immobilization plates is provided with a grid of tool apertures each configured to allow for passage of a tool through the immobilization plate, e.g. a biopsy tool. This allows, for example, taking samples from the body part that has been imaged by inserting a device into the body part through an aperture. For example, a biopsy can be taken from a breast by inserting a hollow needle into the breast directly after imaging the breast. Not only can said tumour candidate or region be imaged accurately, but a biopsy can be taken accurately and fast, minimizing discomfort to the person whose breast is being imaged and increasing accuracy of sampling.

In an embodiment, the first collimator assembly is provided with at least one elongated slot perpendicular to the first immobilization plate, which slot(s) is/are alignable with one or more tool apertures in the first immobilization plate allowing for a tool, e.g. a biopsy tool and/or a marker tool, to be passed through a tool aperture and the slot.

In an embodiment, the system further comprises a marker device, that is configured to pass through a tool aperture and the slot and is adapted to mark the body part.

In an embodiment, the system further comprises a biopsy tool, that is configured to pass through a tool aperture and the slot and is adapted to perform a biopsy of the body part.

In an embodiment, the system is configured, e.g. is provided with a programmed controller, to perform an operation wherein in a first imaging step the first collimator assembly is arranged with the second collimator part thereof between the imaging space and the detector,
and wherein, for performing a second imaging step, the first collimator assembly is moved so that the first collimator assembly is arranged with the first collimator part thereof between the imaging space and the detector.

The first invention also relates to a method of gamma imaging of a body part, e.g. of a breast, wherein use is made of a collimator part and/or a gamma camera and/or a system as discussed herein in relation to the first invention.

### Second invention

The second invention relates to a gamma radiation imaging system, for example for scintimammography or for imaging another human body part, e.g. an extremity like an arm or a leg, or for imaging an animal body part.

Known systems for scintimammography relating to the second invention are described, for example, in WO2010008538, WO2010/014001, WO2010120636, US2013/0158389, and US105159456.

The second invention aims to provide an improved system, in particular an improved scintimammography system.

The second invention aims to provide a system which has an increased sensitivity and/or allows for a reduction of the dose of tracer material that is to be applied, commonly injected, to the person in view of the imaging. The second invention also or alternatively aims to reduce the time required for the imaging, e.g. enhancing comfort for the person, and/or increased accuracy of the localization of the tumorous lesion.

The first aspect of the second invention provides a gamma radiation imaging system, for example for scintimammography, according to clause 1 of the second invention.

The system comprises:
- a frame supporting a first immobilization plate and a second immobilization plate, which second immobilization plate is arranged or arrangeable to extend parallel to the first immobilization plate, wherein the first and second immobilization plates define an imaging space between them and are configured to clamp a body part to be imaged, e.g. a breast, between the immobilization plates,

wherein at least the first immobilization plate is provided with a grid of tool apertures each configured to allow for passage of a tool through the first immobilization plate, e.g. a biopsy tool,
   - a first gamma camera configured and arranged to detect gamma radiation emitted from a volume in the imaging space passing through the first immobilization plate,
   - a second gamma camera configured and arranged to detect gamma radiation emitted from a volume in the imaging space passing through the second immobilization plate,
wherein the first gamma camera comprises:
   - a first collimator assembly, which extends in a plane parallel to the first immobilization plate,
   - a first gamma sensitive detector arranged to receive gamma radiation passing through the first collimator assembly,
wherein the second gamma camera comprises:
   - a second collimator assembly, which extends in a plane parallel to the second immobilization plate,
   - a second gamma sensitive detector arranged to receive gamma radiation passing through the second collimator assembly.

In the system the first and the second collimator assemblies are each movable relative to the respective immobilization plate in said plane parallel to the respective immobilization plate in a displacement direction, e.g. in only one displacement direction. For example, the one displacement direction is along the front of a torso of a female human undergoing imaging of the breast, e.g. horizontally or at an oblique path.

Preferably, the system comprises a collimator motion device which is configured to controllably move the collimator assembly of the gamma camera's, preferably each individually, in the displacement direction, preferably only in the one displacement direction.

The first collimator assembly has a first collimator part which comprises a first section and a second section adjoining the first section along a join which extends perpendicular to the displacement direction, wherein a center plane of the first collimator part extends normal to the first collimator part into the imaging space, which center plane includes the join, wherein the first section is provided with inclined first holes at an inclination angle with the center plane, and wherein the second section is provided with inclined second holes at an inclination angle with the center plane and having an opposite sign with respect to the center plane relative to the first holes.

The second collimator assembly has a first collimator part which comprises a first section and a second section adjoining the first section along a join which extends perpendicular to the displacement direction, wherein a center plane of the second collimator part extends normal to the second collimator part into the imaging space, which center plane includes the join, wherein the first section is provided with inclined first holes at an inclination angle with the center plane, and wherein the second section is provided with inclined second holes at an inclination angle with the center plane and having an opposite sign with respect to the center plane relative to the first holes.

In practical embodiments of the first aspect of the second invention, the first part of the first collimator assembly and the first part of the second collimator assembly may be of the same design.

When imaging is performed using the first parts of both collimator assemblies an enhanced sensitivity and accuracy is achieved. For example, an accurate localization may also be achieved when the tumorous lesion is located close to one of the immobilization plates. In this situation, the accuracy of imaging with the first part of one collimator assembly may be reduced due to a relative great distance to the lesion, whilst the accuracy of imaging with the first part of the other collimator assembly has reduced due to the relative proximity of the lesion.

In practical embodiments, the detector of each gamma camera is a position sensitive detector. Such a detector gives information about in which part of the detector the gamma photon has interacted.

The system is, in a preferred embodiment, configured for scintimammography, e.g. with the person standing or sitting when a breast thereof is imaged using the system.

In a practical embodiment, at least one of the immobilization plates and the associated detector, in practice will other components of the gamma camera, is movable in a direction substantially towards the other immobilization plate, e.g. allowing for a breast to be held immobile under compression. In general compression will cause the human body part to remain in place with respect to the immobilization plates and the cameras, so enhancing the quality of the image. Also compression, at least in case of a breast, makes the part to be imaged thinner, and the obtained images more unambiguous.

In a practical embodiment, at least one of the immobilization plates and the associated collimator assembly and/or entire gamma camera is tiltable, e.g. allowing for the immobilization plates to be arranged non-parallel during imaging. However, in practical embodiments, a parallel arrangement of the immobilization plates and the associated collimator assemblies during imaging is preferred.

In a practical embodiment, during imaging, e.g. during one or more imaging step of the imaging process as discussed herein, the gamma camera's including the respective collimator assembly are stationary relative to the imaging space and the body part.

In a practical embodiment, at least one of the immobilization plates is transparent for visible light allowing to see through the plate by the human eye. In practical embodiments, at least the immobilization plate provided with the grid of tool apertures is transparent for visible light.

In a practical embodiment, both collimators assemblies, e.g. also including the second part as discussed below, are formed by one or more collimator plates having parallel main faces.

In practical embodiments, each collimator assembly lies against a face of the respective immobilization plate.

In practical embodiments, the immobilization plates have a thickness of less than 5 millimeters, more preferably between 1 and 3 millimeters. In embodiments, the collimator assemblies rest against the face of the immobilization plate that is facing away from the imaging space, wherein the plate supports the rather thin plate against deforming, e.g. bending, during holding the body part under compression. This cooperation allows for a rather thin plate, even of the perforated first immobilization plate, which enhances the proximity of the collimator assembly to the imaging space and thereby the imaging.

In practical embodiments, the holes in the first and second section of the first collimator parts and the holes in the second collimator parts have a uniform cross-section, e.g. diameter, over their length, e.g. drilled holes. For example, the diameter of the holes lies between 1 mm and 3 mm, e.g. about 1.5 mm. In other embodiments, the holes are square in cross-section or have a honeycomb cell cross-section.

In practical embodiments, as known in the art, there is a multitude of holes in each collimator part, closely spaced from one another.

For example, the grid of tool apertures is a rectangular grid with equidistant spaced tool apertures.

In practical embodiments, the tool apertures are circular, e.g. having a diameter between 2 mm and 4 mm, e.g. about 3 mm.

In a preferred embodiment, the first collimator assembly further comprises a second collimator part, wherein the second collimator part is provided with parallel perpendicular holes which extend parallel to the center plane, so perpendicular to the first immobilization plate, and the second collimator assembly further comprises a second collimator part, wherein the second collimator part is provided with parallel perpendicular holes which extend parallel to the center plane, so perpendicular to the second immobilization plate.

In a practical embodiment, for one or, as preferred, both of the first and second collimator assembly, the second collimator part adjoins the first collimator part in the displacement direction, e.g. so as to form one movable collimator component. It is noted that separate first and second collimator parts may adjoin one another without actually being connected to one another, e.g. the one being pressed against the other. Having an adjoining arrangement of the two collimator parts, e.g. interconnected so that the two parts form one component, is beneficial in view of speed in the imaging process, in view of the component providing support for the rather thin immobilization plate (when present), etc. In another embodiment, for one or both of the first and second collimator assembly, the second collimator part is a separate component from the first collimator part. This, for example, allows for an approach wherein the second collimator parts are first placed in the operative position and used for a first imaging step, and then removed so that the first collimator parts can then be placed in the operative position to perform a second imaging step. This could be done manually, but in an embodiment, the motion device is configured to perform this exchange of collimator parts for each gamma camera.

In embodiments, wherein for one or both of the first and second collimator assembly there is a second collimator part as discussed herein, an attractive imaging method can be performed, e.g. (semi-)automatically, e.g. controlled by a programmed controller. Herein, with immobilization plates being parallel to one another, in a first imaging step, the first and second collimator assemblies are each arranged with the second collimator part thereof between the imaging space and the corresponding detector. This allows to determine the location of a region of interest in the body part, e.g. the tumorous lesion, in a plane parallel to the immobilization plates, so in X, Y coordinates. For performing a second imaging step, the first and second collimator assemblies are moved or reconfigured so that each collimator assembly is arranged with the first collimator part thereof between the imaging space and the corresponding detector. This allows, as outlined above, to localize the region(s) of interest also in Z-coordinates. Preferably, in order to optimally perform the second imaging step, use is made of the localization of the region of interest as has been determined in the first imaging step in X,Y coordinates. The first collimator parts of both collimator assemblies are then positioned such that this region of interest is located in the center plane normal to the join between the first and second sections of each of the first collimator parts. This allows for optimal localization of the region of interest in a direction normal to the immobilization plates, so in Z-direction.

The method wherein first the X,Y coordinates are determined in the first imaging step using the second collimator parts, and then using this localization to arrange the first collimator parts based on the X,Y localization, allows for increase of speed and accuracy of the X,Y,Z localization of the region of interest. This also may allow for reduction of the required dose of tracer material compared to existing methods.

The apertures in the first stabilization plate allow for a tool to be passed there through, e.g. for making a mark on the body part or for a biopsy. This will require that the first gamma camera, or at least a part thereof, is removed/repositioned so as to allow for placement of the tool. For example, the first gamma camera is hinged to the frame and can be tilted away from its operative position. Most preferably, only a part of the first gamma camera including the detector is removed/repositioned, whilst the first collimator assembly remains in place, preferably one or more slots being provided as discussed below.

In embodiments, the first collimator assembly is provided with at least one elongated slot through the first part, which slot extends in the center plane where the first section and the second section adjoin one another. The slot(s) is/are alignable with one or more, e.g. a series of tool apertures in the first immobilization plate allowing for a tool, e.g. a biopsy tool and/or a marker tool, to be passed through a biopsy aperture and the slot.

In practical embodiments, an elongated slot in the first collimator assembly has a width of between 2 mm and 5 mm, e.g. about 3 mm.

In embodiments, the first collimator assembly is provided with at least one elongated slot through the first collimator assembly, which slot extends where the first collimator part adjoins the second collimator part. The slot(s) is/are alignable with one or more tool apertures in the first immobilization plate allowing for a tool, e.g. a biopsy tool and/or a marker tool, to be passed through a biopsy aperture and the slot.

In practical embodiments, the one or more elongated slots for the tool(s) extend through the collimator in a direction perpendicular to the immobilization plate and have a length that extends perpendicular to the displacement direction. In embodiments, multiple elongated slots extend on a common line.

Due to the presence of one or more slots in the first collimator assembly, in combination with the design of the collimator assemblies that allow for quick and accurate localization, a biopsy can be quickly performed whilst the body part remains held between the immobilization plates.

The one or more slots can also be used for localization of the region of interest from which a biopsy is to be taken, as the slot(s) will - when placed directly over the region of interest - cause a peak of gamma radiation on the first detector.

When taking a biopsy with a tool passing through an aperture in the first immobilization plate, possibly also through a slot as discussed herein, at least the second gamma camera can still be operated, e.g. using the first collimator part of the second camera. For example, the second gamma camera can be used to follow the biopsy tool when penetrating into the body part, e.g. the tool having a radiation emitting portion, e.g. in proximity to a tip of the tool, which is visible by operation of the second gamma camera, e.g. using the first collimator part of the second camera.

In an embodiment, both the first and the second immobilization plates, in embodiments thereof also the first and second collimator assemblies thereof, are provided with apertures and possibly slot(s) to allow for passage of a tool. For example, when a region of interest is found which is closer to the second immobilization plate, the biopsy tool can be then introduced from the side of the second immobilization plate instead of from the side of the first immobilization plate, which reduces the penetration depth.

In embodiments, the system further comprises a comprising a marker device, that is configured to pass through a tool aperture and the slot and is adapted to mark the human body part. Marking may be of relevance, for example, when surgery is to be performed. For example, a mark is made where a peak is registered in the radiation emitted from the body part. The mark may facilitate surgical removal of a tumorous lesion.

In embodiments, the mark is a visual mark, e.g. made with ink or the like.

In embodiments, the mark is gamma-radioactive and is penetrated into the body part, allowing the marker to be seen and used in a subsequent scan/image.

When, for example, the mark is made on a breast that is held under compression between the immobilization plates, it may not be helpful to apply a single mark. In this regard, the presence of one or more slot(s) as discussed herein may be of use, as it may allow for making multiple marks in proximity of the localized region of interest, which may further assist the surgeon. For example, in embodiments, the marking can be done both from the side of the first immobilization plate and the second immobilization plate.

In embodiments, also the second immobilization plate is provided with tool apertures. For example, this may allow for marking the body part from two sides, e.g. in practice from above and below. Such marks many be helpful for later operations.

In embodiments, the system further comprises a biopsy tool, that is configured to pass through a tool aperture and the slot and is adapted to perform a biopsy of the human body part.

In a practical embodiment, each set of holes in each of the first and second sections of the first part of the first and of the second collimator assemblies is two-dimensional with side-by-side rows of holes in the section, the holes in each row extending in a plane that is perpendicular to center plane and extends in the displacement direction. When holes of adjoining first and second sections are located in the same plane, the lines of view or axes of these holes will intersect. If these planes of the holes of the first section are offset relative to the planes of the holes of the second section, the lines of view or axes of these holes will cross one another.

In a practical embodiment, all holes in the first section are parallel to one another and all holes in the second section are parallel to one another. Other arrangements of holes in the first collimator part are also envisaged, e.g. with (parallel) holes closer to the center plane being arranged at a greater inclination relative to the center plane than holes more remote from the center plane, so with a variation of the angle of inclination between the holes of a section.

In an embodiment, the first and/or second collimator assembly has a first collimator part composed of a first section and a second section adjoining the first section in the displacement direction, wherein the first section is provided with a set of parallel first inclined holes at a first angle, wherein the second section is provided with a set of parallel second inclined holes at a second angle, e.g. the first and second angles being of same magnitude yet with opposite sign relative to the center plane.

In a practical embodiment, the first and second angles of a first collimator part will the same for one or, more preferably, both collimator assemblies. The latter is preferred in view of increasing sensitivity. It is also possible that the first and second angles of the holes in the respective sections of the first part one collimator assembly differ from the first and second angles of the holes in the respective sections of the first part of the other collimator assembly. This may have benefits in view of localization in direction perpendicular to the immobilization plates, e.g. in view of triangulation of the tumorous lesion.

The first aspect of the second invention also relates to a method of gamma imaging of a human body part, e.g. of a breast, wherein use is made of the system as described herein in relation to the first aspect of the second invention.

For example, preferably with the immobilization plates parallel to one another, in a first imaging step the first and second collimator assembly are each arranged with the second collimator part thereof between the imaging space and the detector, and, for performing a second imaging step, the first and second collimator assemblies are moved so that each collimator assembly is arranged with the first collimator part thereof between the imaging space and the detector. More preferably, in the first imaging step, the location of a region of interest in the human body part is determined in a plane parallel to the first and second immobilization plates, and, for performing the second imaging step, the first and second collimator assemblies are moved so that the region of interest determined in the first imaging step is located in the center plane normal to the join between the first and second sections of the first collimator part of each of the first and second collimator assemblies, wherein the second imaging step is performed to also locate the region of interest in a direction normal to the first and second immobilization plates.

For example, a person, e.g. a radiotherapist, may study the image obtained in the first imaging step and then select one or more regions of interest in said image, e.g. using suitable graphic image processing software in conjunction with a digital version of the image, e.g. that allows to select an item (such as a suspect region in the object) and then automatically create a contour line or other graphical indicator thereof. Then the collimator assemblies can be positioned, possibly automatically based on instructions derived from the selection that has been made, as discussed above.

For example, use is made of a system wherein the first collimator assembly is brought in a position wherein a slot is aligned with one or more tool apertures in the first immobilization plate, and wherein a tool, e.g. a biopsy tool and/or a marker tool, is passed through a tool aperture and the slot, and wherein, for example, part of the first gamma camera is removable and/or repositionable from an operative position thereof to provide space for the tool.

A second aspect of the second invention relates to a gamma radiation imaging system, for example for scintimammography, comprising:
- a frame supporting a first immobilization plate and a second immobilization plate, which second immobilization plate is arranged or arrangeable to extend parallel to the first immobilization plate, wherein the first and second immobilization plates define an imaging space between them and are configured to clamp a body part to be imaged, e.g. a breast, between the immobilization plates,

wherein at least the first immobilization plate is provided with a grid of tool apertures each configured to allow for passage of a tool through the first immobilization plate, e.g. a biopsy tool,
   - a first gamma camera configured and arranged to detect gamma radiation emitted from a volume in the imaging space passing through the first immobilization plate,
   - optionally, a second gamma camera configured and arranged to detect gamma radiation emitted from a volume in the imaging space passing through the second immobilization plate,
wherein the first gamma camera comprises:
   - a first collimator assembly, which extends in a plane parallel to the first immobilization plate,
   - a first gamma sensitive detector arranged to receive gamma radiation passing through the first collimator assembly,
wherein the optional second gamma camera, when present, comprises:
   - a second collimator assembly, which extends in a plane parallel to the second immobilization plate,
   - a second gamma sensitive detector arranged to receive gamma radiation passing through the second collimator assembly,
wherein the first and the second collimator assemblies are each movable relative to the respective immobilization plate in said plane in a displacement direction, preferably by collimator motion device configured to controllably move the collimator assembly in the displacement direction,
wherein the first collimator assembly has a first collimator part comprising a first section and a second section adjoining the first section along a join which extends perpendicular to the displacement direction, wherein a center plane of the first collimator part extends normal to the first collimator part into the imaging space, which center plane includes the join, wherein the first section is provided with inclined first holes at an inclination angle with the center plane, and wherein the second section is provided with inclined second holes at an inclination angle with the center plane and having an opposite sign with respect to the center plane relative to the first holes,
wherein the first collimator assembly is provided with at least one elongated slot through the first collimator part, which slot(s) extends in the center plane where the first section and the second section adjoin one another,
which slot(s) is/are alignable with one or more tool apertures in the first immobilization plate allowing for a tool, e.g. a biopsy tool and/or a marker tool, to be passed through a tool aperture and the slot.

For example, the system of the second aspect of the second invention further comprises a comprising a marker device, that is configured to pass through a tool aperture and the slot and is adapted to mark the body part.

For example, the system of the second aspect of the second invention further comprises a biopsy tool, that is configured to pass through a tool aperture and the slot and is adapted to perform a biopsy of the body part.

The system of the second aspect of the second invention may also comprises one of more details as discussed herein with reference to the first aspect of the second invention, e.g. as discussed in one or more of the (sub)clauses of the second invention.

The second aspect of the second invention also relates to a method of gamma imaging of a human body part, e.g. of a breast, wherein use is made of the system.

### Third invention

The third invention relates to a gamma radiation imaging system, for example for scintimammography or for imaging another human body part, e.g. an extremity like an arm or a leg, or for imaging an animal body part.

Known systems for scintimammography relating to the third invention are described, for example, in WO2010008538, WO2010/014001, WO2010120636, US2013/0158389, and US105159456.

The third invention aims to provide an improved system, in particular an improved scintimammography system.

The third invention aims to provide a system which has an increased sensitivity and/or allows for a reduction of the required dose of tracer material that is to be applied, commonly injected, to the person in view of the imaging. The third invention also or alternatively aims to reduce the time required for the imaging, e.g. enhancing comfort for the person, and/or increased accuracy of the 3D localization of the tumorous lesion.

The third invention provides a gamma radiation imaging system, for example for scintimammography, according to clause 1 of the third invention.

The system, for example for scintimammography, comprises:
- a frame supporting a first immobilization plate and a second immobilization plate, which second immobilization plate is arranged or arrangeable to extend parallel to the first immobilization plate, wherein the first and second immobilization plates define an imaging space between them and are configured to clamp a body part to be imaged, e.g. a breast, between the immobilization plates,
- a first gamma camera configured and arranged to detect gamma radiation emitted from a volume in the imaging space passing through the first immobilization plate,
- a second gamma camera configured and arranged to detect gamma radiation emitted from a volume in the imaging space passing through the second immobilization plate,

wherein the first gamma camera comprises:
   - a first collimator assembly, which extends in a plane parallel to the first immobilization plate,
   - a first gamma sensitive detector arranged to receive gamma radiation passing through the first collimator assembly,
wherein the second gamma camera comprises:
   - a second collimator assembly, which extends in a plane parallel to the second immobilization plate,
   - a second gamma sensitive detector arranged to receive gamma radiation passing through the second collimator assembly,
wherein the first and the second collimator assembly are each movable in said plane in a displacement direction relative to the respective immobilization plate, preferably by collimator motion device configured to controllably move the collimator assembly in the displacement direction,
wherein the first collimator assembly comprises a first collimator part and a second collimator part, wherein the first collimator part is provided with a set of parallel first holes at a first angle relative to a center plane which extends normal to the first collimator assembly into the imaging space and perpendicular to the displacement direction, wherein the second collimator part is provided with a set of parallel second holes at a second angle relative to said center plane and having an opposite sign with respect to the center plane relative to the first angle of the first holes,
wherein the second collimator assembly comprises a first collimator part and a second collimator part, wherein the first collimator part is provided with a set of parallel first holes at a first angle relative to a center plane which extends normal to the second collimator assembly into the imaging space and perpendicular to the displacement direction, wherein the second collimator part is provided with a set of parallel second holes at a second angle relative to said center plane and having an opposite sign with respect to the center plane relative to the first angle of the first holes,
wherein the first and second collimator assemblies are configured to allow for a first imaging configuration wherein both first collimator parts are in operative position for imaging and a second imaging configuration wherein both second collimator parts are in operative position for imaging,
wherein, for each of the first and second collimator assemblies, the first angle differs in magnitude from the second angle, with the second angle being greater than the first angle,
and wherein the first angles of the parallel first holes of the first collimator parts of the first and second collimator assemblies are the same,
and wherein the second angles of the parallel second holes of the second collimator parts of the first and second collimator assemblies are the same.

The system allows to perform an imaging process by first having the first imaging configuration wherein imaging is done with the first collimator parts and the gamma radiation passing through the first holes of both collimator assemblies, preferably with the immobilization plates and collimator assemblies in parallel arrangement.

The first angle may be 0°, so with the first holes perpendicular to the respective collimator assembly, in practical embodiments perpendicular to the respective immobilization plate. However, a non-perpendicular yet near perpendicular orientation of the first holes is preferred, e.g. with the first angle between 5° and 10°.

Generally, this first imaging configuration allows to determine whether or not regions of interest, e.g. tumorous lesions, are present in the body part, e.g. the breast of a female person. Then, e.g. based on the results of the initial imaging in the first configuration, the system may be brought in the second imaging configuration. Herein, imaging is done with the second collimator parts and the gamma radiation passing through the second holes at the second angle, greater in magnitude than the first angle and with opposite sign relative to the imaginary center plane of the collimator assembly. The first imaging step will provide both information whether or not regions of interest are present in the imaged body part as well as a localization thereof in the plane of the immobilization plates, which may be referred to as localization in X, Y directions. The second imaging step in the second configuration will provide localization in Z direction as well, noting that the body part remains clamped when switching between the first and second imaging configurations.

In embodiments, a person, e.g. a medical doctor or technician, may analyze the image obtained when performing a first imaging step in the first imaging configuration and then determine whether or not one or more regions of interest are present in the image, e.g. using suitable graphic image processing software in conjunction with a digital version of the image. If so, the second imaging configuration may be provided and a second imaging step performed in that configuration to obtain the desired 3D localization of the region(s) of interest.

In embodiments, the first collimator parts are embodied such that, in the first imaging configuration, a line of view into the imaging space associated with a first hole of one first collimator part coincides with a line of view into the imaging space associated with a first hole of the other first collimator part. Preferably, this applies for all first holes of both first collimator parts. This allows for effective processing of the image obtained with the gamma camera's, and may be advantageous for image quality, e.g. an increased sensitivity.

In embodiments, the second collimator parts are embodied such that, in the second imaging configuration, a line of view into the imaging space associated with a second hole of one second collimator part coincides with a line of view into the imaging space associated with a second hole of the other second collimator part. Preferably, this applies for all second holes of both second collimator parts. This allows for effective processing of the image obtained with the gamma camera's, and may be advantageous for image quality, e.g. an increased sensitivity.

In embodiments the first angle is between 0° and 10°, wherein the second angle is greater.

In embodiments, the second angle is between 15° and 25°, e.g. with the first angle between 0° and 10°.

In preferred embodiments, the first angle is between 5° and 10°.

In preferred embodiments, the second angle is between 20° and 25°.

In preferred embodiments, for each of the collimator assemblies, the sum of the first and second angles is between 25° and 35°, more preferably 30°. This arrangement allows for accurate localization, also in Z-direction, without undue loss of imaging quality. Preferably, herein the first angle is between 5° and 10°.

In practical embodiments, the first and second holes in the collimator parts have a uniform cross-section, e.g. diameter, over their length, e.g. drilled holes. For example, the diameter of the holes lies between 1 mm and 3 mm, e.g. about 1.5 mm. In other embodiments, the holes are square in cross-section or have a honeycomb cell cross-section.

In practical embodiments, the detector of a gamma camera is a position sensitive detector. Such a detector gives information about in which part of the detector the gamma photon has interacted.

The system is, in a preferred embodiment, configured for scintimammography, e.g. with the person standing or sitting when a breast thereof is imaged using the system.

In an embodiment, at least one of the immobilization plates, in practical embodiments along with the respective collimator assembly and detector, is movable in a direction substantially towards the other plate, e.g. allowing for a breast to be held immobile under compression during imaging both in the first configuration and the second configuration. In general compression will cause the body part to remain in place with respect to the plates and the gamma cameras, so enhancing the quality of the image. Also compression, at least in case of a breast, makes the part to be imaged thinner, and the obtained images more unambiguous.

In a practical embodiment, at least one of the immobilization plates and the associated collimator assembly and/or detector, e.g. the entire gamma camera, is tiltable, e.g. allowing for the immobilization plates to be arranged non-parallel during imaging, yet imaging in parallel orientation both in the first and in the second configuration is preferred.

In a practical embodiment, during imaging, e.g. during each step of the imaging process as discussed herein, the gamma camera's including the respective collimator assembly are stationary relative to the imaging space.

In a practical embodiment, at least one of the immobilization plates is transparent for visible light allowing to see through the plate by the human eye.

In a practical embodiment, both collimators assemblies, e.g. also including the second part as discussed below, are formed by one or more collimator plates having parallel main faces.

In practical embodiments, each collimator assembly lies against a face of the respective immobilization plate.

In practical embodiments, the immobilization plates have a thickness of less than 5 millimeters, more preferably between 1 and 3 millimeters. In embodiments, the collimator assemblies rest against the face of the immobilization plate that is facing away from the imaging space, wherein the plate supports the rather thin plate against deforming, e.g. bending, during holding the body part under compression. This cooperation allows for a rather thin plate, even of the perforated first immobilization plate, which enhances the proximity of the collimator assembly to the imaging space and thereby the imaging.

In embodiments, for each collimator assembly, the second collimator part adjoins, e.g. is connected to, the first collimator part in the displacement direction, wherein the collimator assemblies are movable between positions thereof to selectively provide the first imaging configuration wherein the first collimator parts are in operative position for imaging and the second imaging configuration wherein the second collimator parts are in operative position for imaging. An alternative would be that the first and second collimator parts are separate and exchangeable components, so that after the first imaging the first collimator parts are removed and then replaced by the second collimator parts, possibly manually. The latter is deemed less efficient than the version wherein the collimator parts adjoin one another to form a unitary collimator assembly.

In an embodiment, at least one of the immobilization plate is provided with a grid of tool apertures each configured to allow for passage of a tool, e.g. a biopsy tool or a marking tool, through the immobilization plate. For example, after first and second imaging steps have been done and a region of interest has been 3D localized, without undoing the clamping of the body part, e.g. breast, the tool is used for a biopsy or marking.

In an embodiment, the collimator assembly associated with the immobilization plate having the tool apertures is provided with at least one elongated slot through the collimator assembly and perpendicular to the displacement direction, which slot is alignable with one or more tool apertures in the immobilization plate allowing for a tool, e.g. a biopsy tool and/or a marker tool, to be passed through the slot and a tool aperture. For example, the slot(s) extends where the first collimator part and the second collimator part adjoin one another.

The third invention also relates to a method of gamma imaging of a body part, e.g. of a breast, wherein use is made of the system as described herein in relation to the third invention.

In embodiments, an attractive imaging method can be performed, e.g. (semi-)automatically, e.g. controlled by a programmed controller. Herein, with immobilization plates being parallel to one another, in a first imaging step and in the first imaging configuration the presence and location of a region of interest in the body part, e.g. the tumorous lesion, is determined with the localization primarily in a plane parallel to the immobilization plates, so in X, Y coordinates. For performing a second imaging step, the first and second collimator assemblies are operated to achieve the second imaging configuration. This allows, as outlined above, to perform the second imaging step and to localize the region(s) of interest also in Z-coordinates.

### Fourth invention

The fourth invention relates to a gamma radiation imaging system, for example for scintimammography or for imaging another human body part, e.g. an extremity like an arm or a leg.

Known systems for scintimammography relating to the fourth invention are described, for example, in WO2010008538, WO2010/014001, WO2010120636, US2013/0158389, and US105159456.

The fourth invention aims to provide an improved system, in particular an improved scintimammography system.

The fourth invention aims to provide a system which has an increased sensitivity and/or allows for a reduction of the dose of tracer material that is to be applied, commonly injected, to the person in view of the imaging. The fourth invention also or alternatively aims to reduce the time required for the imaging, e.g. enhancing comfort for the person, and/or increased accuracy of the localization of the region of interest, e.g. a tumorous lesion.

The fourth invention provides a gamma radiation imaging system, for example for scintimammography, according to clause 1 of the fourth invention.

The system comprises:
- a frame supporting a first immobilization plate and a second immobilization plate, e.g. parallel to the first immobilization plate, the plates defining an imaging space between them and configured to clamp a body part to be imaged, e.g. a breast, between the plates,

wherein at least the first immobilization plate is provided with a grid of tool apertures each configured to allow for passage of a tool through the first immobilization plate,
   - a first gamma camera configured and arranged to detect gamma radiation emitted from a volume in the imaging space passing through the first immobilization plate,
wherein the first gamma camera comprises:
   - a first collimator assembly, which extends in a plane parallel to the first immobilization plate,
   - a first gamma sensitive detector arranged to receive gamma radiation passing through the first collimator,
wherein the first collimator assembly slidably rests against a face of the first immobilization plate that is facing away from the imaging space, and which first collimator assembly is movable in said plane relative to the respective immobilization plate in a displacement direction,
wherein the first collimator assembly has a collimator part provided with one or more sets of collimator openings, e.g. holes or pinholes, through which gamma radiation passes from the imaging space to the detector,
wherein the first collimator assembly has a non-collimating support part adjoining the collimator part, which non-collimating support part is configured to pass a tool there through into a tool aperture,
wherein the first collimator assembly is slidable selectively between a position wherein the collimator part is in operative position for imaging and a position wherein the non-collimating support part is in the operative position for use in passing a tool through the support part and a tool aperture.

In a practical embodiment of the fourth invention, the non-collimating support part comprises multiple openings which are open at the face of the first immobilization plate and opposite thereof, and which openings of the non-collimating support part are greater than each of the tool apertures in the first immobilization plate, so that in the operative position a tool can be passed through an aligned opening of the support part and a tool aperture. In a practical embodiment, the non-collimating support part comprises support ribs defining the multiple openings. Due to the openings, e.g. defined between the ribs, the tool has access to a selected aperture in the plate, e.g. to penetrate a biopsy tool into a breast of a female person.

In an embodiment, the non-collimating support part is made of a pierceable material, e.g. of a plastic foam material, e.g. a rigid plastic foam material, that - in use - is to be pierced by the tool that is to be passed through the support part and a tool aperture.

Due to the provision of the non-collimating support part adjoining the collimator part it is possible to keep the body part immobilized when it is desired to perform an activity requiring a tool, e.g. a biopsy tool or a marking tool, after the imaging has been done. The collimator part is then slid away from the operative position and the adjoining non-collimating support part slides into the operative position.

Also, due to the provision of the non-collimating support part adjoining the collimator part, it is possible to embody the immobilization plate rather thin. In practical embodiments, the immobilization plate has a thickness of less than 5 millimeters, more preferably between 1 and 3 millimeters. A thin immobilization plate would, by itself, bend under the loading during clamping of the body part, e.g. when the collimating part would be removed in order to perform an activity involving a tool that is to be passed through a tool aperture in the immobilization plate. Due to the inventive collimator assembly, this bending is countered by the non-collimating support part as the structure thereof provides the desired structural support for the rather thin plate.

The possibility to have a rather thin immobilization plate is, in turn, beneficial for the quality of the imaging process, e.g. as the collimator part can be closer to the body part that is to be imaged compared to an immobilization plate that is thicker in order to absorb the bending load thereon without the benefit of the inventive design. Improved imaging may enhance, for example, detection and/or localization of a region of interest, e.g. a tumorous lesion in the body part.

A further benefit of the inventive design of the fourth invention may be the reduction of the time required for the imaging combined with the follow-up action involving the tool, e.g. enhancing comfort for the person whose body part, e.g. breast, is to be examined.

For example, the rib structure forms a rectangular grate of ribs or a honeycomb arrangement of ribs.

In a practical embodiment, the collimator part and the non-collimating support part of the assembly are connected to one another, e.g. permanently or, as preferred, by a releasable connection, e.g. screws, bolts, magnets, or other releasable connectors. A releasable connection, e.g., allows to provide different combinations of collimator part(s) and non-collimating support part(s). For example, one could combine a non-collimating support part with a selected one of different collimator parts, e.g. collimator parts having a different configurations to provide different imaging properties.

In a practical embodiment, the collimator part and the non-collimating support part are not connected to one another, yet arranged in an adjoining configuration. For example, the non-collimating support part is pushed against the collimator part when the collimator part is to be slid away from the operative position. In an embodiment, the non-collimating support part is configured to be manually moved into the adjoining arrangement, e.g. to manually slide the collimator part away from the operative position as the non-collimating support part is manually slid into the operative position.

In an embodiment, the non-collimating support part is made of plastic, e.g. fibre reinforced plastic, e.g. with carbon fibre reinforcement. For example, the support part is injection molded or 3D printed, e.g. to form the rib structure.

In an embodiment, the system comprises a set of different non-collimating support parts, e.g. having different designs of the openings therein and/or different features configured to interact with one or more specific tools.

In an embodiment, the system further comprises a tool orientation device configured to be releasably mated with the non-collimating support part and to interact with a tool that is to be passed through a tool aperture so as to provide for correct orientation of the tool. The orientation provided by the tool orientation device may comprise one or more of the position of the tool in the plane of the immobilization plate, the angle of the tool relative to the immobilization plate (e.g. the tool orientation device being configured to keep the tool perpendicular to the immobilization plate or at a specific angle). For example, the tool orientation device is configured to be mated with one or more ribs of the non-collimating support part.

For example, the grid of tool apertures is a uniform, e.g. rectangular, grid with equidistant spaced tool apertures.

In practical embodiments, the tool apertures are circular, e.g. having a diameter between 2 mm and 8 mm, e.g. of about 5 mm. In practical embodiments, the tool apertures are closely spaced from one another, e.g. less spacing between apertures than their diameter. The tool apertures may be non-circular.

Preferably, the system comprises a collimator motion device configured to controllably move the collimator assembly in the displacement direction, preferably only in the one displacement direction.

Preferably, the detector is a position sensitive detector. Such a detector gives information about in which part of the detector the gamma photon has interacted.

The system is, in a preferred embodiment, configured for scintimammography, e.g. with the person standing or sitting when a breast thereof is imaged using the system.

In a practical embodiment, at least one of the immobilization plates is movable in a direction substantially towards the other plate, e.g. allowing for a breast to be held immobile under compression. In general compression will cause the human body part to remain in place with respect to the plates and the cameras, so enhancing the quality of the image. Also compression, at least in case of a breast, makes the part to be imaged thinner, and the obtained images more unambiguous.

In a practical embodiment, the immobilization plates are parallel during imaging. In an embodiment, at least one of the immobilization plates and the associated collimator assembly and/or entire gamma camera is tiltable, e.g. allowing for the immobilization plates to be arranged at will parallel or non-parallel during imaging.

In a practical embodiment, during imaging, e.g. during a step of the imaging process as discussed herein, the gamma camera including the respective collimator assembly is stationary relative to the imaging space.

In a practical embodiment, at least one of the immobilization plates is transparent for visible light allowing to see through the plate by the human eye, e.g. the plate having the tool apertures.

In a practical embodiment, the collimators assembly or assemblies is/are formed by one or more plate shaped components having parallel main faces.

In an embodiment, the system further comprises a biopsy tool, that is configured to pass through an aligned opening of the support part and a tool aperture and is adapted to perform a biopsy of the human body part.

In an embodiment, the system further comprises a comprising a marker tool, that is configured to pass through an aligned opening of the support part and a tool aperture and is adapted to mark the human body part. Marking may be of relevance, for example, when surgery is to be performed. For example, a mark is made where a peak is registered in the radiation emitted from the body part. The mark may facilitate surgical removal of a tumorous lesion. In embodiments, the mark is a visual mark, e.g. made with ink or the like. In embodiments, the mark is gamma-radioactive and is penetrated into the human body part, allowing the marker to be seen and used in a subsequent scan/image.

In an embodiment, the system further comprises a second gamma camera configured and arranged to detect gamma radiation emitted from a volume in the imaging space passing through the second immobilization plate, wherein the second gamma camera comprises:
- a second collimator assembly, which extends in a plane parallel to the second immobilization plate,
- a second gamma sensitive detector arranged to receive gamma radiation passing through the second collimator assembly.

In an embodiment, the second collimator assembly is movable in said plane in a displacement direction relative to the second immobilization plate, preferably by collimator motion device configured to controllably move the collimator assembly in the displacement direction,

In an embodiment, the first and/or second collimator assembly comprises a collimator part provided with parallel holes having an axis which extends perpendicular to the immobilization plate.

In an embodiment, the first and/or second collimator assembly comprises a collimator part composed of a first section and a second section adjoining the first section, wherein the first section is provided with a set of parallel first holes having a first axis at a first angle with the immobilization plate, wherein the second section is provided with a set of parallel second holes having a second axis at a second angle with the immobilization plate, wherein the first and second holes are configured such that the first and second axes are located in one or more planes perpendicular to the immobilization plate and extending parallel to the displacement direction, and such that first and second axes intersect or cross in the imaging space.

In an embodiment, the first and/or second collimator assembly comprises both a collimator part with parallel holes perpendicular to the immobilization plate, and a collimator part having another configuration of holes, e.g. holes at an angle, e.g. as discussed in the paragraph above, as well as the non-collimating support part for the first collimator assembly.

The fourth invention also relates to a method of gamma imaging of a human body part, e.g. of a breast, and performing an activity involving a tool, wherein use is made of the system as described herein in relation to the fourth invention.

The inventions will now be explained with reference to the drawings. In the drawings:
Fig. 1 shows schematically an embodiment of a system according to the first invention during a first imaging step, here for scintimammography of a female breast,
Fig. 2 shows schematically in cross-section an example of a collimator part according to the first invention,
Fig. 3 shows schematically an embodiment of a system according to the second invention during a first imaging step,
Fig. 4 shows the system of Figure 3 from another angle,
Fig. 5 shows the system of Figure 3 in frontal or coronal plane view,
Fig. 6 shows the system of Figure 3 during a second imaging step,
Fig. 7 shows the system of Figure 3 during performance of a biopsy,
Fig. 8 shows in a frontal view schematically an embodiment of a system according to the third invention in the second imaging configuration during a second imaging step,
Fig. 9 shows the system of Figure 8 in the second imaging configuration,
Fig. 10 shows the system of Figure 9 from another angle,
Fig. 11 shows schematically an embodiment of a system according to the fourth invention during performance of a biopsy as a follow-up after an imaging process using the system,
Fig. 12 shows the system of Figure 11 from another angle,
Fig. 13 shows the system of Figure 11 from yet another angle,
Fig. 14 shows the system of Figure 11 from yet another angle.

### First invention

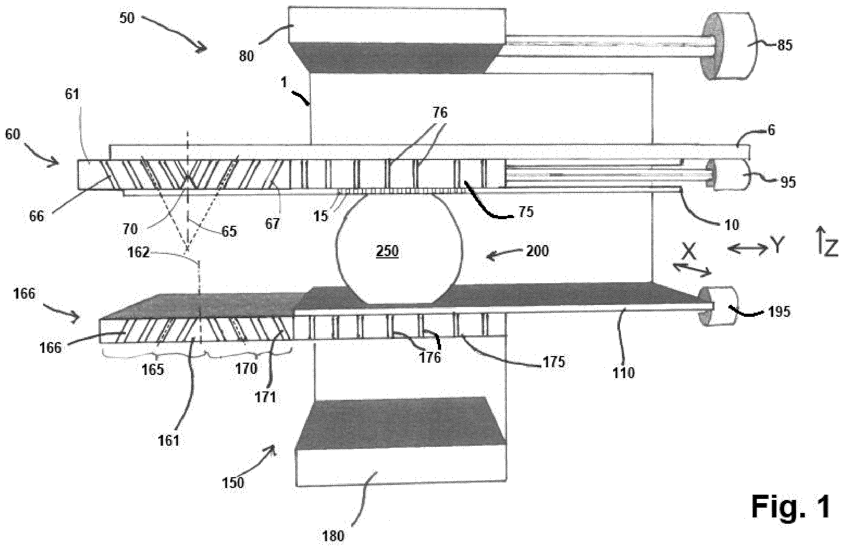

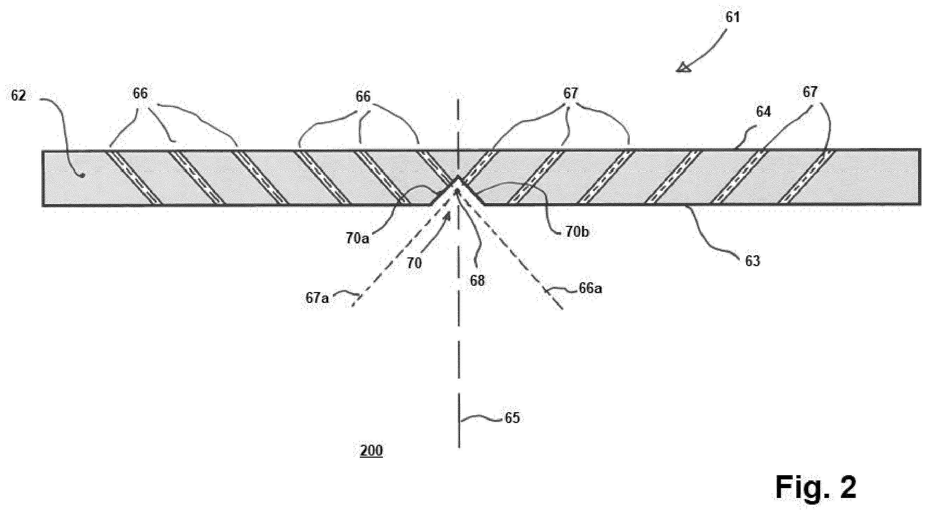

With reference to Figures 1 and 2 an example of a gamma radiation imaging for scintimammography according to the first invention will be discussed.

The system according to the first invention comprises a frame 1, only shown highly schematically in Fig. 1, supporting a first immobilization plate 10 and a second immobilization plate 110 which extends parallel to the first immobilization plate 10. The plates 10, 110 define an imaging space 200 between them for one breast 250 of a female human person, e.g. standing or sitting.

The system is configured to clamp the breast to be imaged between the plates 10, 110.

In a practical embodiment, the plates 10, 110 are made of plastic material, e.g. visually transparent plastic.

The first immobilization plate 10 is provided with a 2D-grid of tool apertures 15, which are each configured to allow for passage of a tool through the first immobilization plate 10. There may a multitude of such apertures, e.g. apertures being spaced closely to one another, e.g. closer than their individual diameter.

The system further comprises a first gamma camera 50 which is configured and arranged to detect gamma radiation emitted from the breast 250 in the imaging space passing through the first immobilization plate 10.

The system further comprises a second gamma camera 150 which is configured and arranged to detect gamma radiation emitted from the breast 250 in the imaging space passing through the second immobilization plate 110.

The first gamma camera 50 comprises:
- a first collimator assembly 60, which extends in a plane parallel to the first immobilization plate 10,
- a first gamma sensitive detector 80 arranged to receive gamma radiation passing through the first collimator assembly 60.

The second gamma camera 150 comprises:
- a second collimator assembly 160, which extends in a plane parallel to the second immobilization plate 110,
- a second gamma sensitive detector 180 arranged to receive gamma radiation passing through the second collimator assembly 160.

In Fig. 1, the detectors are shown to be rather far from the corresponding collimator assembly. This is done for reason of clarity of the Figs. 1-2. In practical embodiments, the detector is in close proximity to the corresponding detector.

The first and the second collimator assemblies 60, 160 are each movable in the plane of the corresponding plate 10, 110 in a displacement direction Y relative to the respective immobilization plate.

Each collimator assembly 60, 160 is linearly driven in Y-direction by a corresponding collimator motion device 95, 195 which configured to controllably move the collimator assembly in the displacement direction Y.

The X-direction extends in the plane of the plates 10, 110, perpendicular to the Y-direction.

As shown, the first collimator assembly 60 has a first collimator part 61 according to the invention which is shown in more detail in figure 2.

The first collimator comprises a plate 62 with a planar incident surface 63 at a front side and a rear surface 64 at a rear side. Here, as preferred, the surfaces 63, 64 are parallel to one another, defining a thickness of the plate.

A virtual normal plane 65 extends perpendicular to the plate in the center thereof.

Multiple inclined first holes 66 extend through the plate from a position on the rear surface 64 on a first side of the normal plane 65.

Multiple inclined second holes 67 extend through the plate from a position on the rear surface on a second side of the normal plane 65.

The first and second holes 66, 67 each have a uniform cross-section, e.g. diameter, over the length thereof.

It is noted that in practical embodiments of the first invention, there is a multitude of holes 66, 67, closely spaced from one another. The limited number of holes in the drawings are shown to increase clarity of Figures 1-2.

Each of the first holes 66 and second holes 67 has a sight line extending along the hole from the rear surface 64 to the planar incident surface 63 and outwards beyond the planar incident surface into imaging space 200 where the body part 250 to be imaged is located.

The first collimator part 61 is provided with an elongated recess 70 at the front side which is recessed relative to the planar incident surface 63 and which extends in the normal plane 65.

As shown, at least one first hole 66 and at least one second hole 67 adjoin the elongated recess 70, so have an opening at the end of the hole that adjoins the recess 70.

In the embodiment shown, the elongated recess 70 forms a groove, here a V-shaped groove in the front side of the collimator part. Here the V-shaped groove is symmetrical relative to the normal plane 65.

As is illustrated in Figure 2, a first sight line 66a of at least one first hole 66 crosses the normal plane 65 on a first crossing position and a second sight line 67a of at least one second hole 67 crosses the normal plane 65 on a second crossing position. As shown, for example, these crossing points coincide and are located on the normal plane, here indicated with reference numeral 68.

As is illustrated, for at least one first hole and for at least one second hole the respective first crossing position and second crossing position, here the common point 68, is positioned inward of the planar incident surface 63, here in the elongated recess or groove 70.

In this example, the first sight line 66a of at least one first hole 66 intersects the second sight line 67a of at least one second hole 67 at a point of intersection 68 which is located in the elongated recess 70. As shown, the point of intersection 68 is, preferably, positioned on the normal plane 65.

As follows from Figure 2 the sight lines of the first and second holes in proximity of the normal plane 65 provide an enhanced field of view, in particular close to the collimator part 61. As explained, this enhanced field of view allows for more effective imaging of regions of interest in the body part that are located close to the collimator part 61 during imaging. This, for example, allows for improved detection of tumorous lesion(s) close to the skin.

As shown, by way of example, the groove 70 is delimited by a first recess surface 70a and a second recess surface 70b at the first side and second side of the normal plane, respectively. The first recess surface 70a and the second recess surface 70b are flat surfaces as preferred.

A first hole 66 defines an opening in the first recess surface 70a, a second hole 67 defines an opening in the second recess surface 70b. As shown, the first sight line 66a is parallel to the second recess surface 70b, and the second sight line 67a is parallel to the first recess surface 70a. A more diverging arrangement of the surfaces 70a, b is not practical as it would not further enhance the field of view and would further reduce the thickness of the plate locally.

The second collimator assembly has a first collimator part 161 of the prior art design in relation to the first invention. This is done to allow for a better understanding of the inventive concept as implemented in the first collimator part 61. The enhanced field of view as explained above is absent in this prior art design of collimator part 161.

The collimator part 161 is embodied as a plate comprising a planar incident surface at a front side and a rear surface at a rear side. A virtual normal plane 162 extends perpendicular to the plate at a join between a first section 165 of the plate and a second section 170 of the plate. In the first section multiple inclined parallel first holes 166 extend through the plate from a position on the rear surface on a first side of the normal plane. The first holes 166 each have an opening in the planar incident surface. In the second section the multiple inclined parallel second holes 171 extend through the plate from a position on the rear surface on a second side of the normal plane. The second holes 171 each have an opening in the planar incident surface. Each of the first and second holes 166, 171 has a sight line extending along the hole from the rear surface to the planar incident surface and outwards beyond the planar incident surface into the imaging space 200 wherein the body part 250 to be imaged is located. Gamma radiation passes from the body part through the first and second holes onto detector 180 of the gamma camera 150 when an image is made. In the known collimator part 161 all openings of the first holes 166 are located on the one side of the normal plane and in the planar incident surface. All openings of the second holes 171 are located on the other side of the normal plane and in the planar incident surface. Such a collimator part is known as a dual slant hole collimator.

It will be appreciated that for the collimator part 161 the crossing points where a sight line crosses the plane 162 all lie outward of the collimator part 161, effectively in the imaging space 200. As explained, a drawback thereof is that the imaging of the portion of the body part 250 close to the immobilization plate 110 is not satisfactory.

In a preferred embodiment of the system according to the first invention, the collimator part 161 is also of the design as shown for first collimator part 61.

As shown by way of example, all first holes 66 are parallel to one another and all second holes 67 are parallel to one another. As explained, this is not a necessity in the context of the first invention. The holes 66, 67 could also, for example, be arranged in a fan beam arrangement.

As preferred, the first collimator assembly 60 further comprises a second collimator part 75. The second collimator part is provided with parallel third perpendicular holes 76 which extend parallel to the normal plane 65, so perpendicular to the first immobilization plate 10.

The second collimator assembly further comprises a second collimator part 175. The second collimator part is provided with parallel third perpendicular holes 176 which extend parallel to the normal plane 65, so perpendicular to the second immobilization plate 110.

In both of the first and the second collimator assembly 60, 160, the second collimator part 75, 175 adjoins the first collimator part 60, 160 in the displacement direction Y.

The collimator assemblies 60, 160 here each are embodied as one component that is slidable in Y-direction along a side of the respective immobilization plate 10, 110 that faces away from the imaging space 200. So, as shown, the first and second parts of each assembly are connected to one another. In another embodiment, these parts adjoin one another without being interconnected.

For example, where the first collimator part 61 adjoins the second collimator part 75 a linear array of elongated slots 90 may be present through the first collimator, the slot(s) being perpendicular to the assembly and the immobilization plate. The line of slots is alignable with one or more tool apertures 15 in the first immobilization plate 10 allowing for a tool, e.g. a biopsy tool and/or a marker tool, to be passed through a tool aperture and the slot.

In embodiments, the system further comprises a comprising a marker device, that is configured to pass through a tool aperture 15 and the slot 90 and is adapted to mark the breast.

As shown in Fig. 1 in a first imaging step the first and second collimator assemblies 60, 160 are each arranged with the second collimator part 75, 175 thereof between the imaging space 200 and the detector 80, 180. As the holes 76, 176 are perpendicular to the plates 10, 110, this generally allows for localization of regions of interest, e.g. a tumorous lesion, in the X,Y plane, so parallel to the plates 10, 110.

For performing a second imaging step, the first and second collimator assemblies 60, 160 are moved by the respective drives 95, 195 to the second imaging configuration, so that each collimator assembly 60, 160 is arranged with the first collimator part 61, 161 thereof between the imaging space 200 and the respective detector 80, 180.

Preferably, the X,Y location of a region of interest in the breast which has been determined in the first imaging step is used for positioning the collimator parts 61, 161 for performing the second imaging step. Herein, the first and second collimator assemblies 61, 161 are moved so that the region of interest determined in the first imaging step is located in the normal plane of the first collimator part of each of the first and second collimator assemblies.

Due to the sets of holes, here parallel slant holes with opposite signs in each of the collimator parts 61, 161, the second imaging step is performed to also, or in particular, locate the region of interest in a direction normal to the first and second immobilization plates 10, 110, which is referred to as the Z-direction.

For example, a person, e.g. a medical doctor or technician, may study the image obtained in the first imaging step and then select one or more regions of interest in said image, e.g. using suitable graphic image processing software in conjunction with a digital version of the image, e.g. that allows to select an item (such as a suspect region in the object) and then create a contour line or other graphical indicator thereof, e.g. a localization of maximum emitted gamma radiation. Then the collimator assemblies 60, 160 can be positioned, possibly automatically based on instructions derived from the selection that has been made, as discussed above.

When it is determined that a biopsy of the region of interest is desired, the breast 250 remains immobilized by the plates 10, 110. The slot(s) 90 are then aligned with the region and with one or more apertures 15 in the plate 10. Part of the first gamma camera 50, at least including the detector 80 in practice also including a housing between the detector 80 and the collimator assembly 60, is repositioned away from the operative position thereof to provide space for the tool 300. The tool 300 is then passes through the slot 90 and an aperture 15 so as to penetrate into the breast 250.

Repositioning of the first gamma camera 50, at least the detector 80, in order to provide access for the tool is done by camera drive 85.

The plates 10, 110 may be fairly thin as the collimator assemblies 60, 160, here effectively embodied as collimator plates 60, 160, remain in contact with the plates 10, 110 and provide support against bending of the plates 10, 110 due to the compression of the breast. The collimator plates 60, 160 are guided by guide rails 5, 6 of the frame of the system.

### Second invention

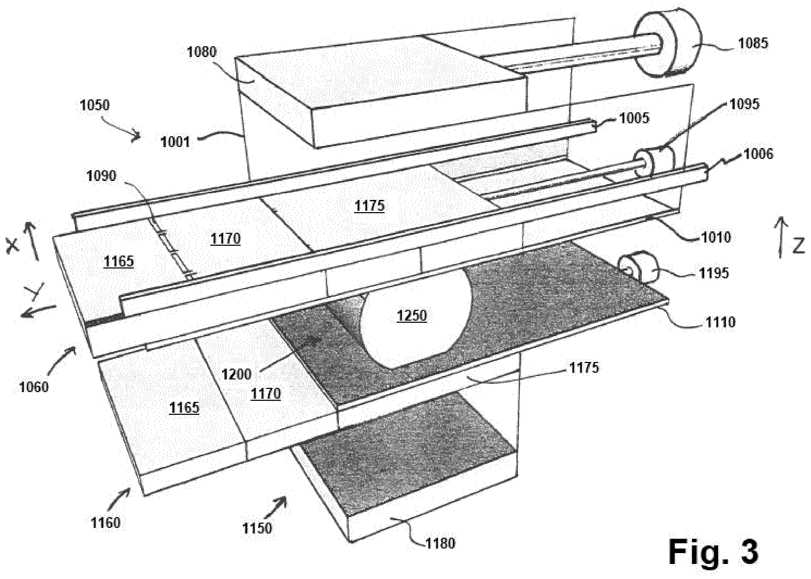

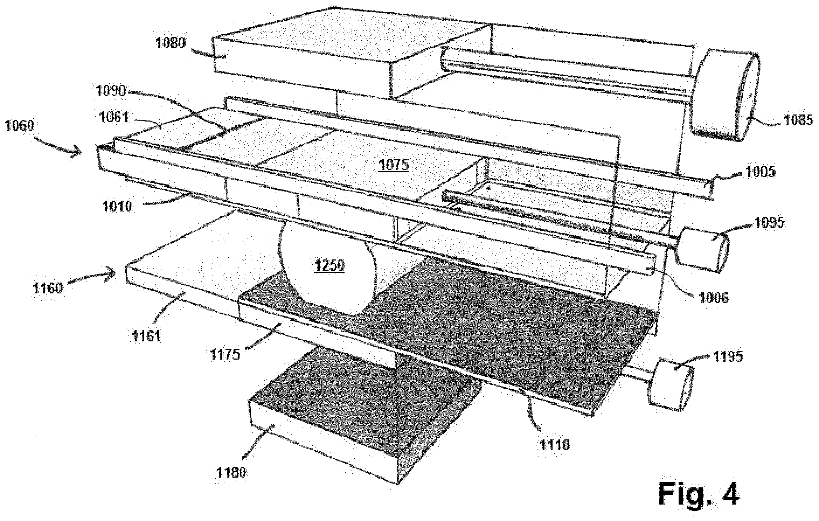

With reference to Figures 3 - 7 an example of a gamma radiation imaging for scintimammography according to the second invention will be discussed.

The system according to the second invention comprises a frame 1001, only shown highly schematically in Figs. 3-7, supporting a first immobilization plate 1010 and a second immobilization plate 1110 which extends parallel to the first immobilization plate 1010. The plates 1010, 1110 define an imaging space 1200 between them for one breast 1250 of a female human person, e.g. standing or sitting.

The system is configured to clamp the breast to be imaged between the plates 1010, 1110.

In a practical embodiment, the plates 1010, 1110 are made of plastic material, e.g. visually transparent plastic.

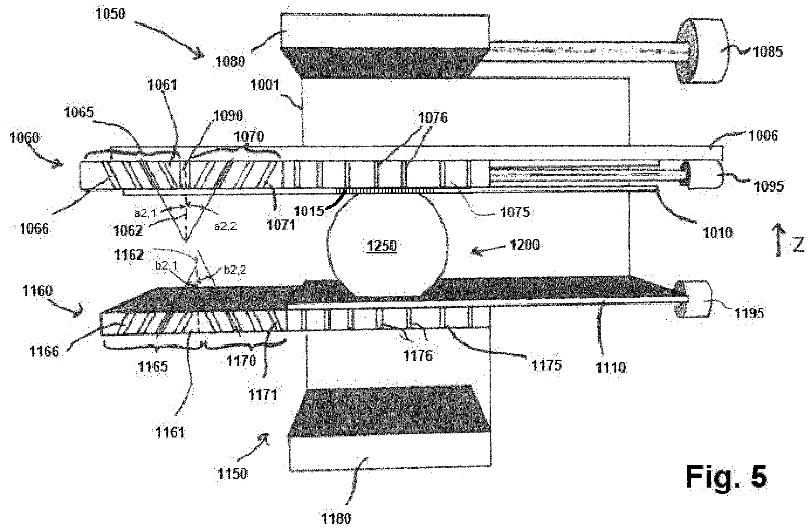

The first immobilization plate 1010 is provided with a 2D-grid of tool apertures 1015, see Fig. 5, which are each configured to allow for passage of a tool 1300 through the first immobilization plate 1010. There may a multitude of such apertures, e.g. apertures being spaced closely to one another, e.g. closer than their individual diameter.

The system further comprises a first gamma camera 1050 which is configured and arranged to detect gamma radiation emitted from the breast 1250 in the imaging space passing through the first immobilization plate 1010.

The system further comprises a second gamma camera 1150 which is configured and arranged to detect gamma radiation emitted from the breast 1250 in the imaging space passing through the second immobilization plate 1110.

The first gamma camera 1050 comprises:
- a first collimator assembly 1060, which extends in a plane parallel to the first immobilization plate 1010,
- a first gamma sensitive detector 1080 arranged to receive gamma radiation passing through the first collimator assembly 1060.

The second gamma camera 1150 comprises:
- a second collimator assembly 1160, which extends in a plane parallel to the second immobilization plate 1110,
- a second gamma sensitive detector 1180 arranged to receive gamma radiation passing through the second collimator assembly 1160.

In Figs. 3-7, the detectors are shown to be rather far from the corresponding collimator assembly. This is done for reason of clarity of the figures. In practical embodiments, the detector is in close proximity to the corresponding detector.

The first and the second collimator assemblies 1060, 1160 are each movable in the plane of the corresponding plate 1010, 1110 in a displacement direction Y relative to the respective immobilization plate.

Each collimator assembly 1060, 1160 is linearly driven in Y-direction by a corresponding collimator motion device 1095, 1195 which configured to controllably move the collimator assembly in the displacement direction Y.

The X-direction extends in the plane of the plates 1010, 1110, perpendicular to the Y-direction.

As shown, the first collimator assembly 1060 has a first collimator part 1061 composed of a first section 1065 and a second section 1070 adjoining the first section 1065 in the displacement direction Y along a join which extends perpendicular to the displacement direction.

An imaginary center plane 1062 associated with the part 1061 extends normal to the part 1061 into the imaging space and includes the join.

As shown by way of example, the first section 1065 is provided with a set of parallel first inclined holes 1066 at a first angle a2,1 with the center plane 1062.

The second section 1070 is provided with a set of parallel second inclined holes 1071 at a second angle a2,2 with the center plane 1062. The angle of these holes 1071 has assign opposite with respective to the center plane relative to the holes 1066.

For example, the first and second holes 1066, 1071 are arranged in rows and extend in planes that are perpendicular to the center plane 1062 and extend parallel to the displacement direction Y.

Generally, the lines of view through the first holes and of the second holes may intersect or cross in the imaging space 1200 seen in X-direction.

At least some of the lines of view through first holes and lines of view through second holes intersect the center plane 1062 in the imaging space 1200.

The second collimator assembly has a first collimator part 1161 composed of a first section 1165 and a second section 1170 adjoining the first section 1165 in the displacement direction Y.

An imaginary center plane 1162 associated with the part 1161 extends normal to the part 1161 into the imaging space and includes the join.

As shown by way of example, the first section 1165 is provided with a set of parallel first inclined holes 1166 at a first angle b2,1 with the center plane 1162.

The second section 1170 is provided with a set of parallel second inclined holes 1171 at a second angle b2,2 with the center plane 1162. The angle of these holes 1171 has a sign opposite with respective to the center plane relative to the holes 1166.

For example, the first and second holes 1166, 1171 are arranged in rows and extend in planes that are perpendicular to the center plane 1162 and extend parallel to the displacement direction Y.

The number of holes shown is far less than in practical embodiments of the collimator parts. This is done for reason of clarity.

The first collimator assembly 1060 further comprises a second collimator part 1075. The second collimator part is provided with parallel third perpendicular holes 1076 which extend parallel to the center plane 1062, so perpendicular to the first immobilization plate 1010.

The second collimator assembly further comprises a second collimator part 1175. The second collimator part is provided with parallel third perpendicular holes 1176 which extend parallel to the center plane 1162, so perpendicular to the second immobilization plate 1110.

In both of the first and the second collimator assembly 1060, 1160, the second collimator part 1075, 1175 adjoins the first collimator part 1060, 1160 in the displacement direction Y.

The collimator assemblies 1060, 1160 here each are embodied as one component that is slidable in Y-direction along a side of the respective immobilization plate 1010, 1110 that faces away from the imaging space 1200. So, as shown, the first and second parts of each assembly are connected to one another. In another embodiment, these parts adjoin one another without being interconnected.

The first collimator assembly is provided with a linear array of elongated slots 1090 through the first collimator, which slots 1090 extend in the center plane 1061 where the first section 1065 and the second section 1070 adjoin one another in the first part 1061.

As shown, the line of slots 1090 is alignable with one or more tool apertures in the first immobilization plate 1010 allowing for a tool, e.g. a biopsy tool 1300 and/or a marker tool, to be passed through a tool aperture and the slot 1090.

In embodiments, the system further comprises a comprising a marker device, that is configured to pass through a tool aperture and the slot 1090 and is adapted to mark the breast.

As shown in Figs. 3, 4, and 5, in a first imaging step the first and second collimator assemblies 1060, 1160 are each arranged with the second collimator part 1075, 1175 thereof between the imaging space 1200 and the detector 1080, 1180. As the holes 1076, 1176 are perpendicular to the plates 1010, 1110, this generally allows for localization of regions of interest, e.g. a tumorous lesion, in the X,Y plane, so parallel to the plates 1010, 1110.

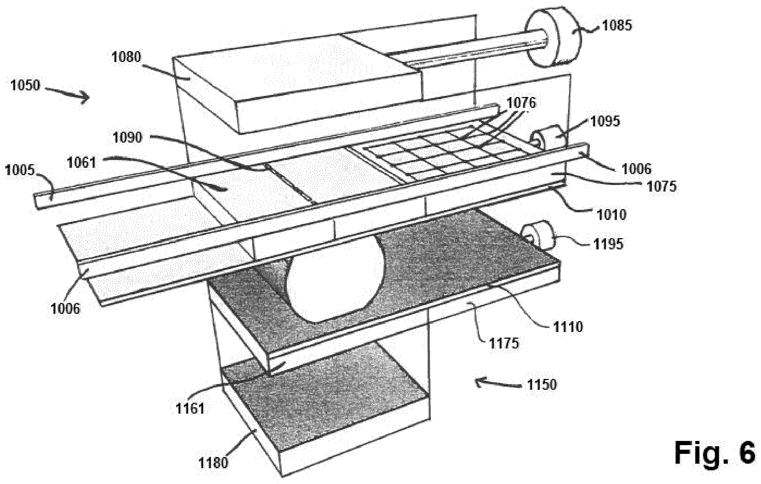

For performing a second imaging step, the first and second collimator assemblies 1060, 1160 are moved by the respective drives 1095, 1195 to the configuration of Fig. 6, so that each collimator assembly 1060, 1160 is arranged with the first collimator part 1061, 1161 thereof between the imaging space 1200 and the respective detector 1080, 1180.

Preferably, the X,Y location of a region of interest in the breast which has been determined in the first imaging step is used for positioning the collimator parts 1061, 1161 for performing the second imaging step. Herein, the first and second collimator assemblies 1061, 1161 are moved so that the region of interest determined in the first imaging step is located in a plane normal to the join, here where the slots 1090, 1190 are located, between the first and second sections of the first collimator part of each of the first and second collimator assemblies.

Due to the sets of parallel slant holes with opposite signs in each of the collimator parts 1061, 1161, the second imaging step is performed to also, or in particular, locate the region of interest in a direction normal to the first and second immobilization plates 1010, 1110, which is referred to as the Z-direction.

For example, a person, e.g. a radiotherapist, may study the image obtained in the first step and then select one or more regions of interest in said image, e.g. using suitable graphic image processing software in conjunction with a digital version of the image, e.g. that allows to select an item (such as a suspect region in the object) and then create a contour line or other graphical indicator thereof, e.g. a localization of maximum emitted gamma radiation. Then the collimator assemblies 1060, 1160 can be positioned, possibly automatically based on instructions derived from the selection that has been made, as discussed above.

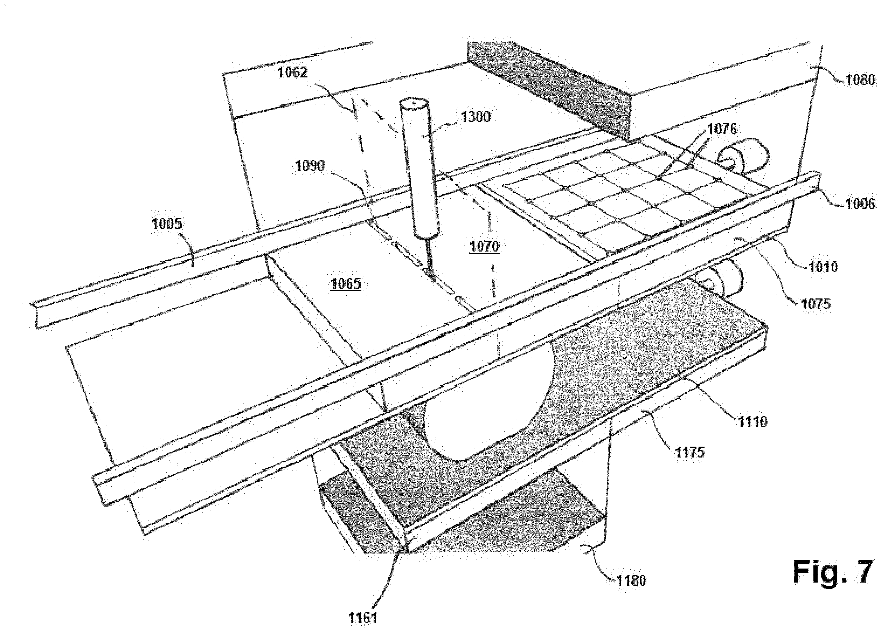

When it is determined that a biopsy of the region of interest is desired, the breast 1250 remains immobilized by the plates 1010, 1110. The slot(s) 1090 are then aligned with the region and with one or more apertures in the plate 1010, as shown in Fig. 7. Part of the first gamma camera 1050, at least including the detector 1080 in practice also including a housing between the detector 1080 and the collimator assembly 1060, is repositioned away from the operative position thereof to provide space for the tool 1300. The tool 1300 is then passes through the slot 1090 and an aperture so as to penetrate into the breast 1250.

Repositioning of the first gamma camera 1050, at least the detector 1080, in order to provide access for the tool 1300 is done by camera drive 1085.

The plates 1010, 1110 may be fairly thin as the collimator assemblies 1060, 1160, here effectively embodied as collimator plates 1060, 1160, remain in contact with the plates 1010, 1110 and provide support against bending of the plates 1010, 1110 due to the compression of the breast. The collimator plates 1060, 1160 are guided by guide rails 1005, 1006 of the frame of the system.

### Third invention

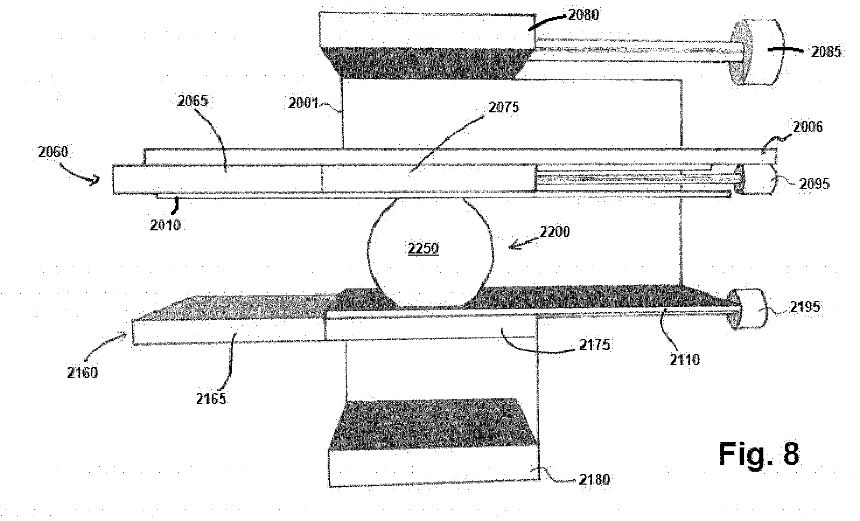

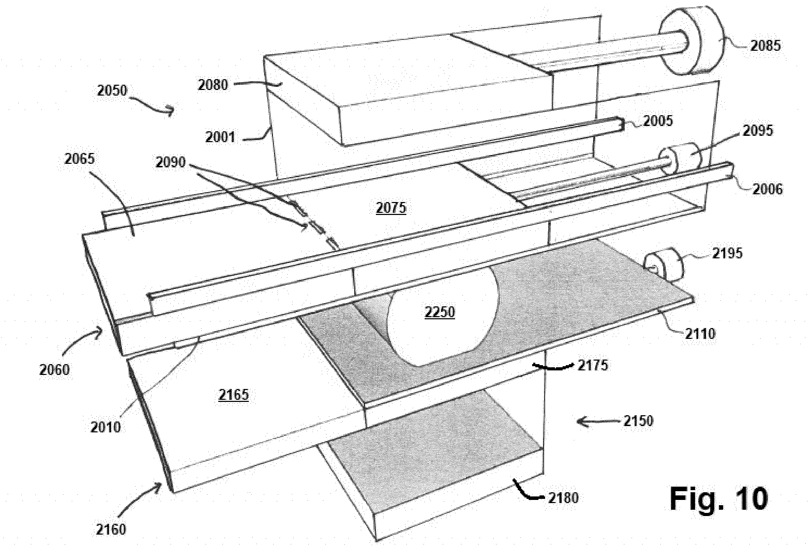

With reference to Figures 8 - 10 an example of a gamma radiation imaging for scintimammography according to the third invention will be discussed.

The system according to the third invention comprises a frame 2001, only shown highly schematically in Figs. 8-10, supporting a first immobilization plate 2010 and a second immobilization plate 2110 which here extends parallel to the first immobilization plate 2010. The plates 2010, 2110 define an imaging space 2200 between them for one breast 2250 of a female human person, e.g. standing or sitting.

The system is configured to clamp the breast to be imaged between the plates 2010, 2110.

In a practical embodiment, the plates 2010, 2110 are made of plastic material.

The first immobilization plate 2010 is provided with a 2D-grid of tool apertures 2015, which are each configured to allow for passage of a tool through the first immobilization plate 2010.

The system further comprises a first gamma camera 2050 which is configured and arranged to detect gamma radiation emitted from the breast 2250 in the imaging space passing through the first immobilization plate 2010.

The system further comprises a second gamma camera 2150 which is configured and arranged to detect gamma radiation emitted from the breast 2250 in the imaging space passing through the second immobilization plate 2110.

The first gamma camera 2050 comprises:
- a first collimator assembly 2060, which extends in a plane parallel to the first immobilization plate 2010,
- a first gamma sensitive detector 2080 arranged to receive gamma radiation passing through the first collimator assembly 2060.

The second gamma camera 2150 comprises:
- a second collimator assembly 2160, which extends in a plane parallel to the second immobilization plate 2110,
- a second gamma sensitive detector 2180 arranged to receive gamma radiation passing through the second collimator assembly 2160.

In Figs. 8-10, the detectors are shown to be rather far from the corresponding collimator assembly. This is done for reason of clarity of the figures. In practical embodiments, the detector is in close proximity to the corresponding detector.

The first and the second collimator assemblies 2060, 2160 are each movable in the plane of the corresponding plate 2010, 2110 in a displacement direction Y relative to the respective immobilization plate. As shown, the plates 2010, 2110 are only movable in the one displacement direction Y. In practice, e.g. when performing imaging of a female breast, so one displacement direction is across the front side of the torso of the person whose breast is imaged, e.g. the Y direction being horizontal or oblique.

The collimator assembly 2060 is guided by guide rails 2005, 2006 of the frame of the system in said Y direction.

Each collimator assembly 2060, 2160 is linearly driven in Y-direction by a corresponding collimator motion device 2095, 2195 which configured to controllably move the collimator assembly in the displacement direction Y.

The X-direction extends in the plane of the plates 2010, 2110, perpendicular to the Y-direction. So, when performing imaging of a female breast, the X direction is generally perpendicular to the front of the torso.

As shown, the first collimator assembly 2060 slidably rests against a face of the first immobilization plate 2010 that is facing away from the imaging space 2200.

The first collimator assembly 2060 comprises a first collimator part 2065 and a second collimator part 2075, wherein the first collimator part is provided with a set of parallel first holes 2066 at a first angle a3,1 relative to a center plane 2062 which extends normal, perpendicular, to the first collimator assembly into the imaging space 2200 and perpendicular to the displacement direction Y. The second collimator part 2075 is provided with a set of parallel second holes 2076 at a second angle a3,2 relative to said center plane 2062 and having an opposite sign with respect to the center plane relative to the first angle a3,1 of the first holes 2066.

It is illustrated that the first collimator part 2065 and a second collimator part 2075 adjoin one another, there is no intermediate collimator part.

It is illustrated that first collimator part 2065 and a second collimator part 2075 each only have the first holes and the second holes, respectively.

The second collimator assembly 2160 comprises a first collimator part 165 and a second collimator part 2175, wherein the first collimator part 2165 is provided with a set of parallel first holes 2166 at a first angle a3,1 relative to a center plane 2162 which extends normal, perpendicular, to the second collimator assembly 2160 into the imaging space 2200 and perpendicular to the displacement direction Y. The second collimator part 2175 is provided with a set of parallel second holes 2176 at a second angle a3,2 relative to said center plane 2162 and having an opposite sign with respect to the center plane 2162 relative to the first angle of the first holes 2166.

It is illustrated that the first collimator part 2165 and a second collimator part 2175 adjoin one another, there is no intermediate collimator part.

It is illustrated that first collimator part 2165 and a second collimator part 2175 each only have the first holes and the second holes, respectively.

The collimator assemblies 2060, 2160 are configured to allow for a first imaging configuration, not shown, wherein only the first collimator parts 2065, 2165 are in operative position for imaging and a second imaging configuration, see Figs. 8, 9, and 10, wherein only the second collimator parts 2075, 2175 are in operative position for imaging.

For each of the first and second collimator assemblies 2060, 2160, the first angle a3,1 differs in magnitude from the respective second angle a3,2, with the second angle a3,2 being greater than the first angle a3,1.

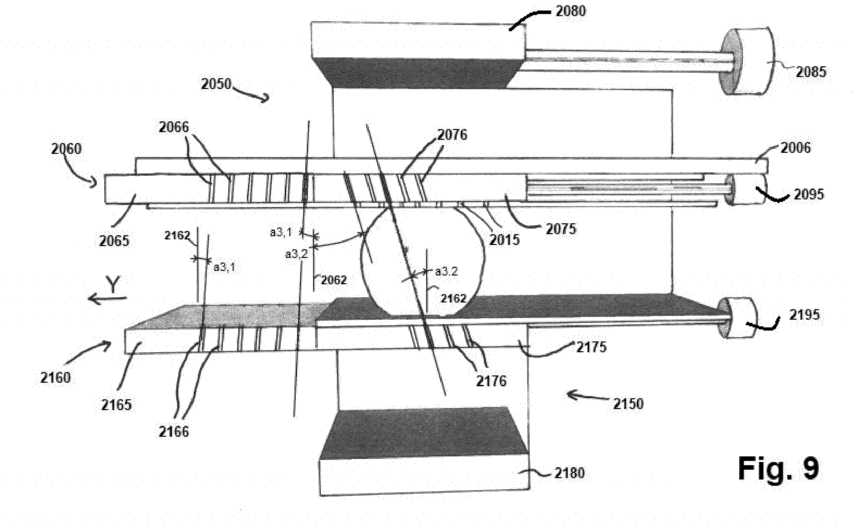

As shown schematically in Fig. 9, the first collimator parts 2065, 2165 are embodied such that, in the first imaging configuration, a line of view into the imaging space associated with a first hole 2066 of one first collimator part 2065 coincides with a line of view into the imaging space associated with a first hole 2166 of the other first collimator part 2165. As explained, this preferably applies for all first holes 2066, 2166 or at least to a majority of the first holes.

As shown schematically, the second collimator parts 2075, 2175 are embodied such that, in the second imaging configuration, a line of view into the imaging space associated with a second hole 2076 of one second collimator part 2075 coincides with a line of view into the imaging space associated with a second hole 2176 of the other second collimator part 2176. As explained, this preferably applies for all second holes 2076, 2176 or at least to a majority of the second holes.

The first angle a3,1 is between 0° and 10°, and the second angle a3,2 is greater, yet with an opposite sign relative to the respective center plane.

For example, the second angle a3,2 is between 15° and 25°.

For example, the first angle a3,1 is between 5° and 10°, wherein the second angle a3,2 is between 20° and 25°.

Preferably, as shown - for each collimator assembly 2060, 2160 - the sum of the first and second angles is between 25° and 35°, preferably 30°.

As shown here, it is preferred for each collimator assembly that the second collimator part 2075, 2175 adjoins and is connected to the first collimator part 2065, 2165 in the displacement direction Y allowing them to be moved in unison by the corresponding drive 2095, 2195.

The drives 2095, 2195 can be operated to move the collimator assemblies 2060, 2160 between positions thereof to selectively provide the first imaging configuration wherein only the first collimator parts 2065, 2165 are in operative position for imaging and the second imaging configuration wherein only the second collimator parts 2075, 2175 are in operative position for imaging. The body part remains immobilized between the plates 2010, 2110 during both imaging steps and during the transition from the first imaging configuration to the second imaging configuration.

The first immobilization plate 2010 is, as preferred, provided with a grid of tool apertures 2015 each configured to allow for passage of a tool, e.g. a biopsy tool or a marking tool, through the immobilization plate. There may a multitude of such apertures, e.g. apertures being spaced closely to one another, e.g. closer than their individual diameter. For example, the grid of tool apertures is a rectangular grid with equidistant spaced tool apertures. In practical embodiments, the tool apertures are circular, e.g. having a diameter between 2 mm and 4 mm, e.g. about 3 mm.

The collimator assembly 2060 associated with the immobilization plate 2010 having the tool apertures is provided with at least one elongated slot 2090 through the collimator assembly, which slot(s) 2090 extends here where the first collimator part 2065 and the second collimator part 2075 section adjoin one another and perpendicular to the displacement direction Y. The slot 2090 is alignable with one or more tool apertures in the immobilization plate 2010 allowing for a tool, e.g. a biopsy tool and/or a marker tool, to be passed through the slot 2090 and a tool aperture.

### Fourth invention

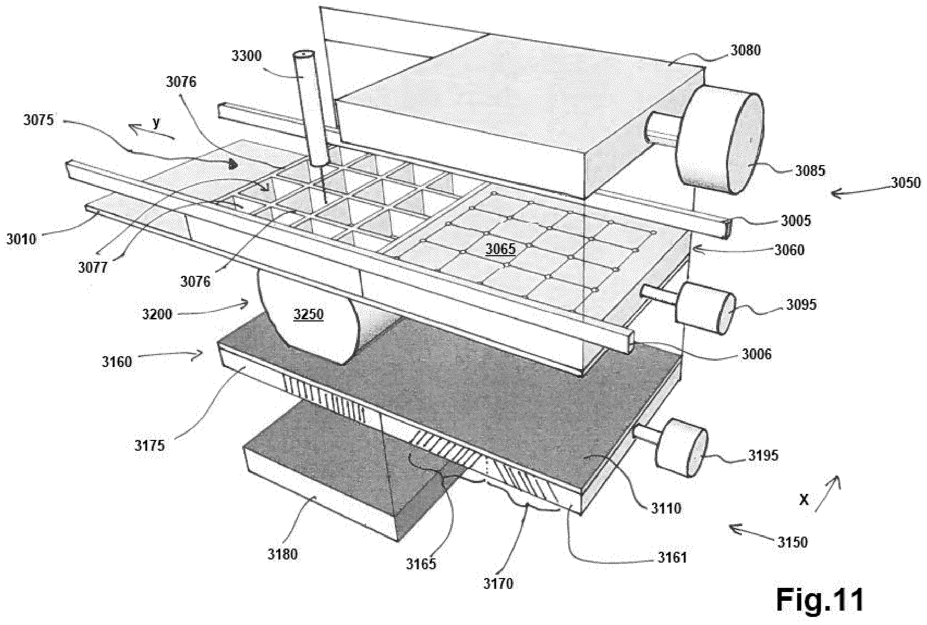

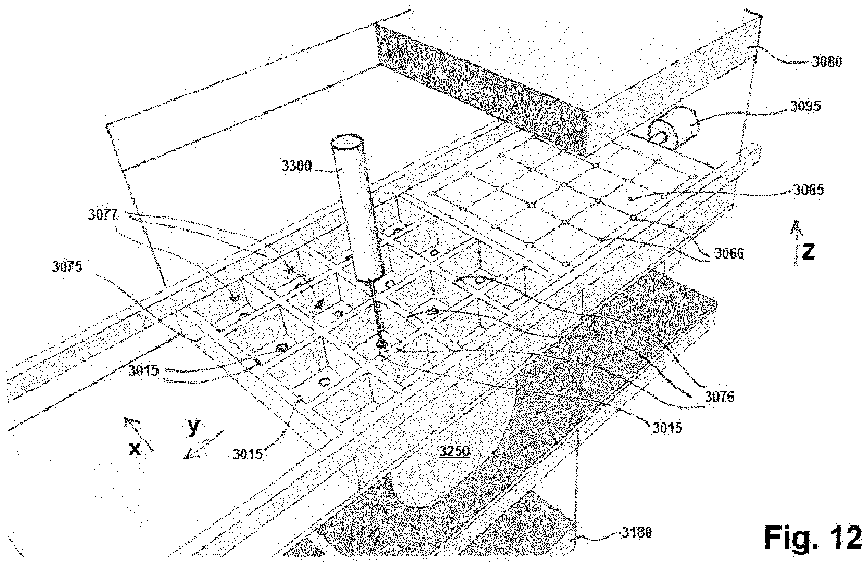

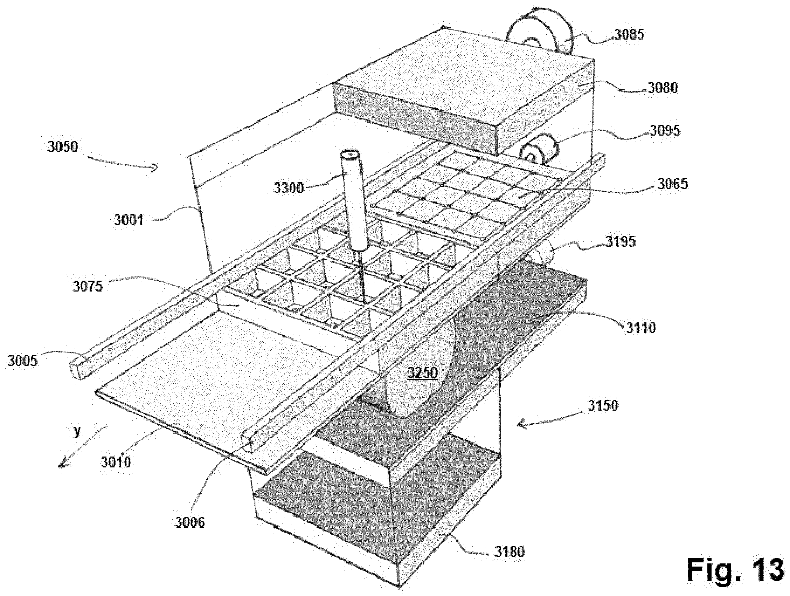

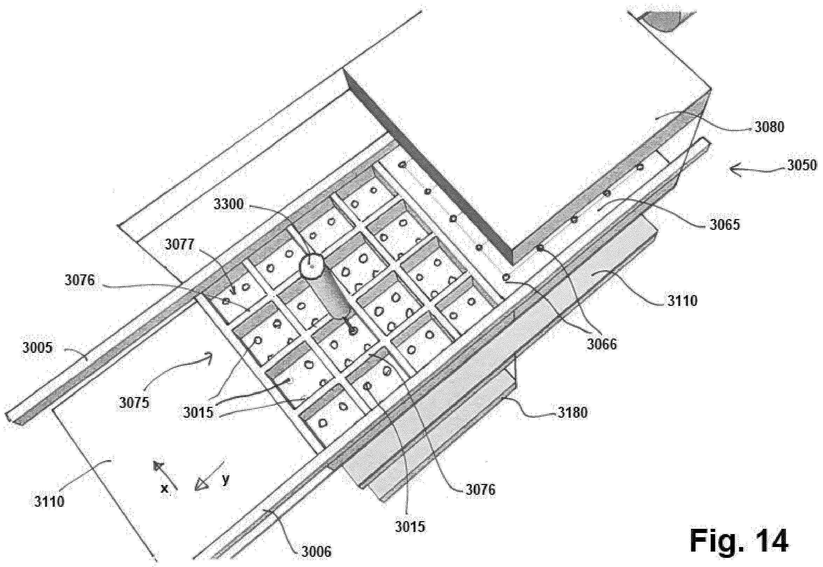

With reference to Figures 11-14 an example of a gamma radiation imaging for scintimammography according to the fourth invention will be discussed.

The system according to the fourth invention comprises a frame 3001, only shown highly schematically in Figs. 11-14, supporting a first immobilization plate 3010 and a second immobilization plate 3110 which here extends parallel to the first immobilization plate 3010. The plates 3010, 3110 define an imaging space 3200 between them for one breast 3250 (shown schematic) of a female human person, e.g. standing or sitting.

The system is configured to clamp the breast to be imaged between the plates 3010, 3110.

In a practical embodiment, the plates 3010, 3110 are made of plastic material, e.g. one or both plates of a visually transparent plastic material.

The first immobilization plate 3010 is provided with a 2D-grid of tool apertures 3015, which are each configured to allow for passage of a tool 3300 through the first immobilization plate 3010. In practical embodiments, the plate 3010 has a multitude of apertures 3015, closely spaced from one another. In practical embodiments, e.g. in view of the tool(s) that is to be passed through an aperture, the diameter of each aperture 3015 may be several millimeters, e.g. between 3 and 8 millimeters, e.g. about 5 - 6 millimeters.

The system further comprises a first gamma camera 3050 which is configured and arranged to detect gamma radiation emitted from the breast 3250 in the imaging space passing through the first immobilization plate 3010.

The system further comprises a second gamma camera 3150 which is configured and arranged to detect gamma radiation emitted from the breast 3250 in the imaging space passing through the second immobilization plate 3110.

The first gamma camera 3050 comprises:
- a first collimator assembly 3060, which extends in a plane parallel to the first immobilization plate 3010,
- a first gamma sensitive detector 3080 arranged to receive gamma radiation passing through the first collimator assembly 3060.

The second gamma camera 3150 comprises:
- a second collimator assembly 3160, which extends in a plane parallel to the second immobilization plate 3110,
- a second gamma sensitive detector 3180 arranged to receive gamma radiation passing through the second collimator assembly 3160.

It is noted that in Figs. 11-14 the detectors 3080, 3180 are shown to be quite far away from the associated collimator assembly. This is done for clarity of the figures, in practical embodiments the detectors 3080, 3180 are in close proximity to the associated collimator assembly.

The first and the second collimator assemblies 3060, 3160 are each movable in the plane of the corresponding plate 3010, 3110 in a displacement direction Y relative to the respective immobilization plate.

The collimator assembly 3060 is guided by guide rails 3005, 3006 of the frame of the system.

Each collimator assembly 3060, 3160 is linearly driven in Y-direction by a corresponding collimator motion device 3095, 3195 which configured to controllably move the collimator assembly in the displacement direction Y.

The X-direction extends in the plane of the plates 3010, 3110, perpendicular to the Y-direction.

As shown, the first collimator assembly 3060 slidably rests against a face of the first immobilization plate 3010 that is facing away from the imaging space 3200.

The first collimator assembly has a collimator part 3065 which is provided with one or more sets of collimator openings, e.g. holes 3066 or pinholes, through which gamma radiation passes from the imaging space to the detector 3080.

The first collimator assembly has a non-collimating support part 3075 adjoining the collimator part 3065.

The non-collimating support part 3075, e.g. made of plastic, comprises support ribs 3076, here a grate of ribs 3076, here a rectangular grate of ribs 3076. The ribs 3076 define openings 3077 which are open at the face of the first immobilization plate 3010 as well as opposite thereof.

In embodiments, the ribs 3076 have a height perpendicular to the plate 3010 and a width that is less than the height.

The cross-section of the ribs 3075 may be uniform over the length of the ribs, but other configurations are also envisaged. The ribs 3076 may be rectilinear, as shown, but also could be curved, undulating, etc.

The openings 3077 of the non-collimating support part 3075 are greater than each of the tool apertures 3015 in the first immobilization plate 3010. In practical embodiments, one opening 3077 is greater in size than a subgroup of multiple apertures in the plate 3010. For example, one opening 3077 is 40 millimeters by 40 millimeters, seen from above.

The first collimator assembly 3060 is slidable selectively between a position wherein the collimator part 3065 is in operative position for imaging of the breast with the first gamma camera 3050 and a position, shown in Figs. 11, 12, and 13, wherein the non-collimating support part 3075 is in the operative position thereof for use in passing the tool 3300 through an aligned opening 3077 of the support part 3075 and a tool aperture 3015 in the plate 3010. In use, the breast 3250 is clamped between the plates 3010, 3110, e.g. by moving the plate 3010 along with the assembly 3060, the detector 3080, and rest of camera 3050 towards the other plate 3110.

Due to the provision of the non-collimating support part 3075 adjoining the collimator part, it is possible to embody the immobilization plate 3010 rather thin.

In practical embodiments, the immobilization plate 3010, e.g. the plastic plate 3010, has a thickness of less than 5 millimeters, more preferably between 1 and 3 millimeters, e.g. 2 - 3 millimeters.

A thin immobilization plate 3010 would, by itself, bend under the loading during clamping of the body part, here breast 3250, e.g. when the collimating part would be removed in order to perform an activity involving the tool 3300 that is to be passed through a selected tool aperture 3015 in the immobilization plate 3010. Due to the inventive structure of the collimator assembly, this bending is countered by the non-collimating support part 3075 as the rib structure thereof provides the desired structural support for the rather thin plate 3010. This support is provided throughout the step of sliding the assembly such that the collimator part is slid away from the operative position and the support part 3075 in slid into the operative position.

Due to the openings defined between the ribs of part 3075, the tool 3300 has access to a selected aperture 3015 in the plate 3010, e.g. to penetrate a biopsy tool into a breast 3250 of a female person.

In practical embodiments, one or more friction reducing features may be present in the system to reduce friction between the plate 3010 and the assembly 3060 during sliding. For example, a Teflon coating is provided, pressurized air or another lubricant is used between the plate 3010 and the assembly 3060, etc.

The second collimator assembly 3160 has a first collimator part 3161 composed of a first section 3165 and a second section 3170 adjoining the first section 3165 in the displacement direction Y.

In this example, the first section 3165 is provided with a set of parallel first holes having a first axis at a first angle with the second immobilization plate 3110. The second section 3170 is provided with a set of parallel second holes having a second axis at a second angle with the second immobilization plate 3110. The first and second holes are configured such that the first and second axes are located in one or more planes perpendicular to the second immobilization plate 3110 and extending parallel to the displacement direction Y, and such that first and second axes intersect or cross in the imaging space 3200 seen in X-direction.

The second collimator assembly further comprises, as an example, a second collimator part 3175, wherein the second collimator part is provided with parallel third holes 3176 having a third axis which extends perpendicular to the second immobilization plate 3110.

The collimator assemblies 3060, 3160 here each are embodied as one component that is slidable in Y-direction along a side of the respective immobilization plate 3010, 3110 that faces away from the imaging space 3200.

When it is determined that a biopsy of the region of interest is desired, the breast 3250 remains immobilized by the plates 3010, 3110.

Part of the first gamma camera 3050, at least including the detector 3080 in practice also including a housing between the detector 3080 and the collimator assembly 3060, is repositioned away from the operative position thereof to provide space for the tool 3300 and access to the apertures 3015. The tool 3300 is then passed through an opening in the part 3075 and an aperture 3015 so as to penetrate into the breast 3250, make a mark on the breast, etc.

Repositioning of the first gamma camera 3050, at least the detector 3080, in order to provide access for the tool 3300 is done here by camera drive 3085.

In an embodiment, not shown, the system further comprises a tool orientation device configured to be releasably mated with the non-collimating support part and to interact with the tool 3300 that is to be passed through a tool aperture so as to provide for correct orientation of the tool. The orientation provided by the tool orientation device may comprise one or more of the position of the tool 3300 in the plane of the immobilization plate, the angle of the tool 3300 relative to the immobilization plate (e.g. the tool orientation device being configured to keep the tool perpendicular to the immobilization plate or at a specific angle). For example, the tool orientation device is configured to be mated with one or more ribs of the non-collimating support part 3075. For example, the tool orientation device has a mating portion that fits into one or more openings of the part 3075 to mate the device with the part 3075. During taking of a biopsy with tool 3300, the second gamma camera 3150 can, if desired, be operational, e.g. to monitor penetration of the tool into the part 3250. For example, part 3161 of the assembly 3160 is used during taking of a biopsy, e.g. the join between sections 3165 and 3170 being aligned with the location of the biopsy tool 3300 to optimize monitoring of penetration depth, e.g. the biopsy tool, e.g. the tip thereof, being provided with a tracer emitting gamma radiation.

### Second invention

Clauses relating to the second invention:
Clause 1. A gamma radiation imaging system, for example for scintimammography, comprising:
   - a frame supporting a first immobilization plate and a second immobilization plate, which second immobilization plate is arranged or arrangeable to extend parallel to the first immobilization plate, wherein the first and second immobilization plates define an imaging space between them and are configured to clamp a body part to be imaged, e.g. a breast, between the immobilization plates,

   wherein at least the first immobilization plate is provided with a grid of tool apertures each configured to allow for passage of a tool through the first immobilization plate, e.g. a biopsy tool,
      - a first gamma camera configured and arranged to detect gamma radiation emitted from a volume in the imaging space passing through the first immobilization plate,
      - a second gamma camera configured and arranged to detect gamma radiation emitted from a volume in the imaging space passing through the second immobilization plate,
   wherein the first gamma camera comprises:
      - a first collimator assembly, which extends in a plane parallel to the first immobilization plate,
      - a first gamma sensitive detector arranged to receive gamma radiation passing through the first collimator assembly,
   wherein the second gamma camera comprises:
      - a second collimator assembly, which extends in a plane parallel to the second immobilization plate,
      - a second gamma sensitive detector arranged to receive gamma radiation passing through the second collimator assembly,
   wherein the first and the second collimator assemblies are each movable relative to the respective immobilization plate in said plane in a displacement direction, preferably by collimator motion device configured to controllably move the collimator assembly in the displacement direction,
   wherein the first collimator assembly has a first collimator part comprising a first section and a second section adjoining the first section along a join which extends perpendicular to the displacement direction, wherein a center plane of the first collimator part extends normal to the first collimator part into the imaging space, which center plane includes the join, wherein the first section is provided with inclined first holes at an inclination angle with the center plane, and wherein the second section is provided with inclined second holes at an inclination angle with the center plane and having an opposite sign with respect to the center plane relative to the first holes,
   wherein the second collimator assembly has a first collimator part comprising a first section and a second section adjoining the first section along a join which extends perpendicular to the displacement direction, wherein a center plane of the second collimator part extends normal to the second collimator part into the imaging space, which center plane includes the join, wherein the first section is provided with inclined first holes at an inclination angle with the center plane, and wherein the second section is provided with inclined second holes at an inclination angle with the center plane and having an opposite sign with respect to the center plane relative to the first holes.
Clause 2. System according to clause 1, wherein the first collimator assembly further comprises a second collimator part, wherein the second collimator part is provided with parallel perpendicular holes which extend parallel to the center plane, and
   wherein the second collimator assembly further comprises a second collimator part, wherein the second collimator part is provided with parallel perpendicular holes which extend parallel to the center plane.
Clause 3. System according to clause 2, wherein - for one or both of the first and the second collimator assembly - the second collimator part adjoins the first collimator part in the displacement direction.
Clause 4. System according to any one or more of the clauses 1-3, wherein the first collimator assembly is provided with at least one elongated slot through the first collimator part, which slot(s) extends in the center plane where the first section and the second section adjoin one another,
   which slot(s) is/are alignable with one or more tool apertures in the first immobilization plate allowing for a tool, e.g. a biopsy tool and/or a marker tool, to be passed through a tool aperture and the slot.
Clause 5. System according to any one or more of the clauses 1-4, wherein the system further comprises a comprising a marker device, that is configured to pass through a tool aperture and the slot and is adapted to mark the body part.
Clause 6. System according to any one or more of the clauses 1-5, wherein the system further comprises a biopsy tool, that is configured to pass through a tool aperture and the slot and is adapted to perform a biopsy of the body part.
Clause 7. System according to any one of more of the clauses 1-6, wherein the system is configured, e.g. is provided with a programmed controller, to perform an operation wherein in a first imaging step the first and second collimator assembly are each arranged with the second collimator part thereof between the imaging space and the detector,
   and wherein, for performing a second imaging step, the first and second collimator assemblies are moved so that each collimator assembly is arranged with the first collimator part thereof between the imaging space and the detector.
Clause 8. A method of gamma imaging of a human body part, e.g. of a breast, wherein use is made of the system according to any one or more of the clauses 1-7.
Clause 9. Method according to clause 8, wherein use is made of a system according to at least clause 2, wherein in a first imaging step the first and second collimator assembly are each arranged with the second collimator part thereof between the imaging space and the detector, and wherein, for performing a second imaging step, the first and second collimator assemblies are moved so that each collimator assembly is arranged with the first collimator part thereof between the imaging space and the detector.
Clause 10. Method according to clause 9, wherein, in the first imaging step, the location of a region of interest in the body part is determined in a plane parallel to the first and second immobilization plates, and wherein, for performing the second imaging step, the first and second collimator assemblies are moved so that the region of interest determined in the first imaging step is located in the center plane normal to the join between the first and second sections of the first collimator part of each of the first and second collimator assemblies, wherein the second imaging step is performed to also locate the region of interest in a direction normal to the first and second immobilization plates.
Clause 11. Method according to any one or more of clauses 8 - 10, wherein use is made of a system according to clause 4, wherein the first collimator assembly is brought in a position wherein a slot is aligned with one or more tool apertures in the first immobilization plate, and wherein a tool, e.g. a biopsy tool and/or a marker tool, is passed through a tool aperture and the slot, and wherein, for example, part of the first gamma camera is removable and/or repositionable from an operative position thereof to provide space for the tool.

### Third invention

Clauses relating to the third invention:
Clause 1. A gamma radiation imaging system, for example for scintimammography, comprising:
   - a frame supporting a first immobilization plate and a second immobilization plate, which second immobilization plate is arranged or arrangeable to extend parallel to the first immobilization plate, wherein the first and second immobilization plates define an imaging space between them and are configured to clamp a body part to be imaged, e.g. a breast, between the immobilization plates,
   - a first gamma camera configured and arranged to detect gamma radiation emitted from a volume in the imaging space passing through the first immobilization plate,
   - a second gamma camera configured and arranged to detect gamma radiation emitted from a volume in the imaging space passing through the second immobilization plate,

   wherein the first gamma camera comprises:
      - a first collimator assembly, which extends in a plane parallel to the first immobilization plate,
      - a first gamma sensitive detector arranged to receive gamma radiation passing through the first collimator assembly,
   wherein the second gamma camera comprises:
      - a second collimator assembly, which extends in a plane parallel to the second immobilization plate,
      - a second gamma sensitive detector arranged to receive gamma radiation passing through the second collimator assembly,
   wherein the first and the second collimator assemblies are each movable relative to the respective immobilization plate in said plane in a displacement direction, preferably by collimator motion device configured to controllably move the collimator assembly in the displacement direction,
   wherein the first collimator assembly comprises a first collimator part and a second collimator part, wherein the first collimator part is provided with a set of parallel first holes at a first angle relative to a center plane which extends normal to the first collimator assembly into the imaging space and perpendicular to the displacement direction, wherein the second collimator part is provided with a set of parallel second holes at a second angle relative to said center plane and having an opposite sign with respect to the center plane relative to the first angle of the first holes,
   wherein the second collimator assembly comprises a first collimator part and a second collimator part, wherein the first collimator part is provided with a set of parallel first holes at a first angle relative to a center plane which extends normal to the second collimator assembly into the imaging space and perpendicular to the displacement direction, wherein the second collimator part is provided with a set of parallel second holes at a second angle relative to said center plane and having an opposite sign with respect to the center plane relative to the first angle of the first holes,
   wherein the first and second collimator assemblies are configured to allow for a first imaging configuration wherein both first collimator parts are in operative position for imaging and a second imaging configuration wherein both second collimator parts are in operative position for imaging,
   wherein, for each of the first and second collimator assemblies, the first angle differs in magnitude from the second angle, with the second angle being greater than the first angle,
   and wherein the first angles of the parallel first holes of the first collimator parts of the first and second collimator assemblies are the same,
   and wherein the second angles of the parallel second holes of the second collimator parts of the first and second collimator assemblies are the same.
Clause 2. System according to clause 1, wherein the first collimator parts are embodied such that, in the first imaging configuration, a line of view into the imaging space associated with a first hole of one first collimator part coincides with a line of view into the imaging space associated with a first hole of the other first collimator part.
Clause 3. System according to clause 2, wherein the first collimator parts are embodied such that, in the first imaging configuration and for all first holes, a line of view into the imaging space associated with a first hole of one first collimator part coincides with a line of view into the imaging space associated with a first hole of the other first collimator part
Clause 4. System according to any one or more of clauses 1 - 3, wherein the second collimator parts are embodied such that, in the second imaging configuration, a line of view into the imaging space associated with a second hole of one second collimator part coincides with a line of view into the imaging space associated with a second hole of the other second collimator part.
Clause 5. System according to clause 4, wherein the second collimator parts are embodied such that, in the second imaging configuration and for all first holes, a line of view into the imaging space associated with a second hole of one second collimator part coincides with a line of view into the imaging space associated with a second hole of the other second collimator part.
Clause 6. System according to any one or more of clauses 1 - 5, wherein the first angle is between 0° and 10°, and wherein the second angle is greater.
Clause 7. System according to any one or more of clauses 1 - 6, wherein the second angle is between 15° and 25°.
Clause 8. System according to any one or more of clauses 1 - 7, wherein the first angle is between 5° and 10°, and wherein the second angle is between 20° and 25°.
Clause 9. System according to any one or more of clauses 1 - 8, wherein - for each collimator assembly - the sum of the first angle and second angle is between 25° and 35°, preferably 30°.
Clause 10. System according to any one or more of clauses 1 - 9, wherein, for each collimator assembly, the second collimator part adjoins and is connected, e.g. releasable, to the first collimator part in the displacement direction,
   and wherein the collimator assemblies are movable between positions thereof to selectively provide the first imaging configuration wherein the first collimator parts are in operative position for imaging and the second imaging configuration wherein the second collimator parts are in operative position for imaging.
Clause 11. System according to any one or more of clauses 1 - 10, wherein at least one or the immobilization plates is provided with a grid of tool apertures each configured to allow for passage of a tool, e.g. a biopsy tool or a marking tool, through the immobilization plate.
Clause 12. System according to clause 11, wherein the collimator assembly associated with the immobilization plate having the tool apertures is provided with at least one elongated slot through the collimator assembly and perpendicular to the displacement direction, which slot is alignable with one or more tool apertures in the immobilization plate allowing for a tool, e.g. a biopsy tool and/or a marker tool, to be passed through the slot and a tool aperture.
Clause 13. System according to any one or more of clauses 1 - 12, wherein the system is configured for scintimammography.
Clause 14. A method of gamma imaging of a human body part, e.g. of a breast, wherein use is made of the system according to any one or more of the clauses 1 - 13.
Clause 15. Method according to clause 14, wherein the image obtained when performing a first imaging step in the first imaging configuration is analyzed and based thereon a determination is made whether or not one or more regions of interest are present in the image, and, if so, the second imaging configuration is provided, and a second imaging step is performed in the second configuration to obtain the desired 3D localization of the region(s) of interest.

### Fourth invention

Clauses relating to the fourth invention:
Clause 1. A gamma radiation imaging system, for example for scintimammography, comprising:
   - a frame supporting a first immobilization plate and a second immobilization plate, e.g. parallel to the first immobilization plate, the immobilization plates defining an imaging space between them and configured to clamp a body part to be imaged, e.g. a breast, between the plates,

   wherein at least the first immobilization plate is provided with a grid of tool apertures each configured to allow for passage of a tool through the first immobilization plate,
      - a first gamma camera configured and arranged to detect gamma radiation emitted from a volume in the imaging space passing through the first immobilization plate,
   wherein the first gamma camera comprises:
      - a first collimator assembly, which extends in a plane parallel to the first immobilization plate,
      - a first gamma sensitive detector arranged to receive gamma radiation passing through the first collimator,
   wherein the first collimator assembly slidably rests against a face of the first immobilization plate that is facing away from the imaging space, and which first collimator assembly is movable in said plane relative to the respective immobilization plate in a displacement direction, preferably by collimator motion device configured to controllably move the collimator assembly,
   wherein the first collimator assembly has a collimator part provided with one or more sets of collimator openings through which gamma radiation passes from the imaging space to the detector,
   wherein the first collimator assembly has a non-collimating support part adjoining the collimator part, which non-collimating support part is configured to pass a tool there through into a tool aperture,
   wherein the first collimator assembly is slidable selectively between a position wherein the collimator part is in operative position for imaging and a position wherein the non-collimating support part is in the operative position for use in passing a tool through the support part and a tool aperture.
Clause 2. System according to clause 1, wherein the non-collimating support part comprises multiple openings which are open at the face of the first immobilization plate and opposite thereof, and which openings of the non-collimating support part are greater than each of the tool apertures in the first immobilization plate, so that in the operative position a tool can be passed through an aligned opening of the support part and a tool aperture.
Clause 3. System according to clause 2, wherein the non-collimating support part comprises support ribs defining the multiple openings.
Clause 4. System according to any one or more of clauses 1 - 3, wherein the non-collimating support part is made of a pierceable material, e.g. of a plastic foam material, e.g. a rigid plastic foam material, that - in use - is to be pierced by the tool that is to be passed through the support part and a tool aperture.
Clause 5. System according to any one or more of clauses 1 - 4, wherein the system further comprises a biopsy tool, that is configured to pass through the support part and a tool aperture and is adapted to perform a biopsy of the human body part.
Clause 6. System according to any one or more of clauses 1 - 5, wherein the system further comprises a comprising a marker tool, that is configured to pass through the support part and a tool aperture and is adapted to mark the human body part.
Clause 7. System according to any one or more of clauses 1-6, wherein the system further comprises a second gamma camera configured and arranged to detect gamma radiation emitted from a volume in the imaging space passing through the second immobilization plate,
   wherein the second gamma camera comprises:
   - a second collimator assembly, which extends in a plane parallel to the second immobilization plate,
   - a second gamma sensitive detector arranged to receive gamma radiation passing through the second collimator assembly.
Clause 8. System according to clause 7, wherein the second collimator assembly is movable in said plane in a displacement direction relative to the second immobilization plate, preferably by collimator motion device configured to controllably move the collimator assembly in the displacement direction,
Clause 9. System according to any one of the clauses 1 - 8, wherein the first and/or second collimator assembly comprises a collimator part provided with parallel holes having an axis which extends perpendicular to the immobilization plate.
Clause 10. System according to any one of the clauses 1 - 9, wherein the first and/or second collimator assembly comprises a collimator part composed of a first section and a second section adjoining the first section, wherein the first section is provided with a set of parallel first holes having a first axis at a first angle with the immobilization plate, wherein the second section is provided with a set of parallel second holes having a second axis at a second angle with the immobilization plate, wherein the first and second holes are configured such that the first and second axes are located in one or more planes perpendicular to the immobilization plate and extending parallel to the displacement direction, and such that first and second axes intersect or cross in the imaging space.
Clause 11. A method of gamma imaging of a human body part, e.g. of a breast, wherein use is made of the system according to any one or more of the clauses 1 - 10.

## Claims

1. Collimator part (61) for collimating gamma radiation in a gamma radiation imaging system, for example for scintimammography, the collimator part comprising:
- a plate comprising a planar incident surface (63) at a front side and a rear surface (64) at a rear side,
- a virtual normal plane (65) perpendicular to the plate,
- multiple inclined first holes (66) extending through the plate from a position on the rear surface on a first side of the normal plane (65),
- multiple inclined second holes (67) extending through the plate from a position on the rear surface on a second side of the normal plane (65),
wherein:
- each of the first and second holes (66, 67) has a sight line (66a, 67a) extending along the hole from the rear surface (64) to the planar incident surface (63) and outwards beyond the planar incident surface,
- a first sight line (66a) of at least one first hole (66) crosses the normal plane (65) on a first crossing position (68),
- a second sight line (67a) of at least one second hole (67) crosses the normal plane on a second crossing position (68),
- for at least one first hole (67) and for at least one second hole (67) the respective first crossing position (68) and second crossing position (68) is positioned inward of the planar incident surface (63).

2. Collimator part according to claim 1, the collimator part is provided with an elongated recess (70) at the front side which is recessed relative to the planar incident surface (63) and which extends in the normal plane, wherein at least one first hole (66) and at least one second hole (67) adjoin the elongated recess (70), wherein for said first hole and second hole the respective first crossing position (68) and second crossing position (68) is positioned in the elongated recess (70), inward of the planar incident surface (63).

3. Collimator part according to claim 2, wherein the elongated recess (70) at the front side is symmetrical relative to the normal plane (65), preferably wherein the elongated recess (70) is a V-shaped groove.

4. Collimator part according to claim 2 or 3, wherein:
- the elongated recess (70) is delimited by a first recess surface (70a) and a second recess surface (70b) at the first side and second side of the normal plane (65), respectively,
wherein, preferably, the first recess surface (70a) and the second recess surface (70b) are flat surfaces,
- a first hole (66) defines an opening in the first recess surface (70a),
- a second hole (67) defines an opening in the second recess surface (70b),
- a first sight line (66a) of the at least one first hole is parallel to the second recess surface (70b),
- a second sight line (67a) of the at least one second hole is parallel to the first recess surface (70a).

5. Collimator part according to any one or more of claims 2 - 4, wherein the first sight line of at least one first hole intersects the second sight line of at least one second hole at a point of intersection (68) which is located in the elongated recess, wherein the point of intersection (68) is, preferably, positioned on the normal plane (65).

6. Collimator part according to any one or more of the preceding claims, wherein the inclined first holes (66) are parallel to one another to form a first parallel slant hole collimator arrangement, and wherein the inclined second holes (67) are parallel to one another to form a second parallel slant hole collimator arrangement,
preferably wherein the sight lines of the inclined first holes (66) are arranged at a first angle with respect to the normal plane and the sight lines of the inclined second holes (67) are arranged at a second angle with respect to the normal plane, wherein the magnitude of the first angle is the same as the magnitude of the second angle.

7. Collimator part according to any one or more of the preceding claims, wherein the first and second holes (66, 67) each have a uniform cross-section, e.g. diameter, over the length thereof.

8. Gamma camera comprising a detector for gamma radiation and a collimator part (61) according to any one or more of the preceding claims.

9. A gamma radiation imaging system, for example for scintimammography, comprising:
- a frame supporting a first immobilization plate (10) and a second immobilization plate (110), which second immobilization plate is arranged or arrangeable to extend parallel to the first immobilization plate, wherein the first and second immobilization plates define an imaging space between them and are configured to clamp a body part to be imaged, e.g. a breast, between the immobilization plates,
- a first gamma camera (50) configured and arranged to detect gamma radiation emitted from a volume in the imaging space passing through the first immobilization plate,
wherein the first gamma camera comprises:
- a first collimator assembly (60) which extends in a plane parallel to the first immobilization plate, which first collimator assembly comprising a first collimator part (61) according to any one or more of claims 1 - 7,
- a first gamma sensitive detector (80) arranged to receive gamma radiation passing through the first collimator assembly.

10. System according to claim 9, wherein the first collimator assembly further comprises a second collimator part (75), wherein the second collimator part is provided with parallel perpendicular holes which extend parallel to the normal plane (65).

11. System according to claim 9 or 10, wherein the system further comprises a second gamma camera (150) configured and arranged to detect gamma radiation emitted from a volume in the imaging space passing through the second immobilization plate (110),
wherein the second gamma camera comprises:
- a second collimator assembly (160), which extends in a plane parallel to the second immobilization plate,
- a second gamma sensitive detector(180) arranged to receive gamma radiation passing through the second collimator assembly.

12. System according to any one or more of claims 9 - 11, wherein the first and/or the second collimator assembly (60, 160) is movable relative to the respective immobilization plate in said plane in a displacement direction, preferably by collimator motion device configured to controllably move the collimator assembly in the displacement direction.

13. System according to any one or more of claims 9 - 12, wherein at least one of the immobilization plates (10) is provided with a grid of tool apertures each configured to allow for passage of a tool through the first immobilization plate, e.g. a biopsy tool,
preferably wherein the first collimator assembly is provided with at least one elongated slot perpendicular to the first immobilization plate (10), which slot(s) is/are alignable with one or more tool apertures in the first immobilization plate allowing for a tool to be passed through a tool aperture and the slot,
preferably wherein the system further comprises a marker device, that is configured to pass through a tool aperture and the slot and is adapted to mark the body part,
preferably wherein the system further comprises a biopsy tool, that is configured to pass through a tool aperture and the slot and is adapted to perform a biopsy of the body part.

14. System according to claim 10, wherein the system is configured, e.g. is provided with a programmed controller, to perform an operation wherein in a first imaging step the first collimator assembly is arranged with the second collimator part (75) thereof between the imaging space and the detector, and wherein, for performing a second imaging step, the first collimator assembly is moved so that the first collimator assembly is arranged with the first collimator part (61) thereof between the imaging space and the detector.

15. Method of gamma imaging of a body part, e.g. of a breast, wherein use is made of a collimator part (61) according to any one or more of the claims 1 - 7, and/or a gamma camera (50) according to claim 8, and/or a system according to any one or more of claims 9 - 14.
